(19) Europäisches Patentamt / European Patent Office / Office européen des brevets

(11) **EP 3 884 266 B1**

(12) **FASCICULE DE BREVET EUROPEEN**

(45) Date de publication et mention
de la délivrance du brevet:
**06.09.2023 Bulletin 2023/36**

(21) Numéro de dépôt: **19831783.6**

(22) Date de dépôt: **18.11.2019**

(51) Classification Internationale des Brevets (IPC):
**G01N 21/55** *(2014.01)* **G01N 21/78** *(2006.01)*
**G01N 33/00** *(2006.01)*

(52) Classification Coopérative des Brevets (CPC):
**G01N 21/55; G01N 21/77; G01N 33/0047;**
G01N 33/0062; G01N 2021/7773

(86) Numéro de dépôt international:
**PCT/FR2019/052736**

(87) Numéro de publication internationale:
**WO 2020/104746 (28.05.2020 Gazette 2020/22)**

(54) **DISPOSITIF OPTIQUE DE DÉTECTION DE COMPOSÉS VOLATILS ET PROCÉDÉ DE DÉTECTION ET DE QUANTIFICATION DE COMPOSÉS VOLATILS ASSOCIÉ**

OPTISCHE VORRICHTUNG ZUR DETEKTION VON FLÜCHTIGEN VERBINDUNGEN UND ZUGEHÖRIGES VERFAHREN ZUR DETEKTION UND QUANTIFIZIERUNG VON FLÜCHTIGEN VERBINDUNGEN

OPTICAL DEVICE FOR DETECTING VOLATILE COMPOUNDS AND ASSOCIATED METHOD FOR DETECTING AND QUANTIFYING VOLATILE COMPOUNDS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.11.2018 FR 1871648**

(43) Date de publication de la demande:
**29.09.2021 Bulletin 2021/39**

(73) Titulaires:
• **Université d'Aix Marseille**
**13007 Marseille 7 (FR)**
• **Centre National de la Recherche Scientifique**
**75016 Paris 16 (FR)**

(72) Inventeur: **GROSSO, David**
**13190 ALLAUCH (FR)**

(74) Mandataire: **INNOV-GROUP**
**310, avenue Berthelot**
**69372 Lyon Cedex 08 (FR)**

(56) Documents cités:
**JP-A- 2016 138 782     US-A1- 2014 021 967
US-B2- 9 007 593**

**Description**

La présente invention concerne le domaine des capteurs, et plus particulièrement des capteurs de composés volatils, tels que des gaz par exemple, ainsi que le domaine de l'utilisation de tels capteurs.

De nos jours, de nombreux polluants peuvent être présents dans une atmosphère, comme par exemple l'intérieur d'une pièce, ou encore l'intérieur d'un habitacle de véhicule automobile. Les polluants sont généralement sous forme gazeuse. Ces gaz peuvent être incolores et inodores, ou encore présents à des seuils trop faibles pour être détectés directement par un utilisateur lorsque celui-ci respire. Or, de tels gaz peuvent être nocifs pour la santé en cas d'exposition régulière, répétée, ou encore pendant de longues durées.

On connaît de l'art antérieur des capteurs configurés pour détecter un élément particulier et en déterminer sa concentration dans une atmosphère à tester, comme cela est par exemple décrit dans le document WO 2014/189758.

D'autre part, on connaît par exemple du document WO 2007/019517, un détecteur de polluants dans un gaz, mettant en oeuvre un four de chromatographie en phase gazeuse afin de permettre une désorption thermique de composés adsorbés sur une couche sélective. Le dispositif décrit dans ce document met également en oeuvre un concentrateur en amont du dispositif de mesure configuré pour concentrer le gaz à analyser avant son entrée dans le dispositif de mesure. De plus, les différents éléments désorbés sont ensuite analysés. Cependant, le détecteur de polluants décrit dans ce document met en oeuvre des éléments consommant de grandes quantités d'énergie et nécessite un dispositif d'analyse afin de déterminer la présence d'un polluant dans ce gaz. Ainsi, le détecteur de polluants décrit dans ce document peut être susceptible d'améliorations.

On connaît également des documents US 2016/0084786, FR 2871573, et de l'article « Planar indium tin oxide heater for improved thermal distribution for métal oxide micromachined gas sensors », Sensors, 2016, 16, 1612 des détecteurs de gaz mettant en oeuvre une couche sensible sur laquelle les composés volatils peuvent s'adsorber. Ces composés adsorbés sont ensuite désorbés par voie thermique et la désorption de ces composés est déterminée par modification d'une propriété physique de cette couche sensible, et notamment sa résistance. Cependant, la détermination de la modification de la résistance peut être compliquée à mettre en oeuvre. De plus, la détermination de la modification de la résistance de cette couche sensible ne permet pas de déterminer la nature chimique du composé désorbé et il est donc nécessaire de faire des analyses complémentaires ultérieures. Les différents détecteurs décrits dans ces documents semblent donc avoir des temps de fonctionnement assez longs afin de déterminer la nature chimique des composés volatils désorbés. De plus, l'utilisation d'une couche ITO comme élément chauffant et une couche sensible à transduction électrique nécessite que ces deux éléments soient séparés par un élément non conducteur. Un tel détecteur de gaz présente donc une structure complexe et coûteuse.

D'autre part, on connaît du document FR 3046244, un détecteur de gaz présentant une couche sensible fixe ou mobile sur laquelle les composés volatils peuvent s'adsorber. L'adsorption ou la désorption de composés volatils sur cette couche sensible est déterminée par la variation d'une propriété physico-chimique de cette couche sensible. La désorption de composés volatils selon ce document se fait par voie thermique. Cependant, ce document précise que la composition de la couche sensible peut être adaptée à la nature du composé volatil que l'on souhaite détecter. Toutefois, ce document ne divulgue ni ne suggère la manière dont la nature chimique des composés volatils une fois désorbés peut être déterminée ou encore leur concentration évaluée.

Le document US2014/021967 A1 décrit un détecteur de gaz présentant un élément réflecteur comprenant une couche sensible transparente et poreuse sur laquelle les composés volatils peuvent s'adsorber. Des mesures de réflexion optique et des mesures de capacitance permettent de déterminer les composés volatils présents dans l'atmosphère à tester et leur concentration.

Le document US9007593 B2 décrit un détecteur de gaz présentant un élément réflecteur comprenant une couche sensible transparente et poreuse sur laquelle les composés volatils peuvent s'adsorber et se désorber en fonction de la température de la couche sensible. En chauffant la couche sensible, des mesures de réflexion optique permettent de déterminer les composés volatils présents dans l'atmosphère à tester selon leurs températures de désorption et leur concentration.

La présente invention a pour objectif de proposer un détecteur permettant de détecter et de quantifier au moins un composé volatil dans une atmosphère à tester qui soit simple d'utilisation et offre des résultats dans un temps amélioré par rapport aux détecteurs de l'art antérieur.

Un autre objectif de la présente invention, différent de l'objectif précédent, est de proposer un détecteur de composés volatils qui soit bon marché.

Un autre objectif de la présente invention, différent des objectifs précédents, est de proposer un détecteur de composés volatils efficace même pour de faibles concentrations en composés volatils dans l'atmosphère à tester.

Un autre objectif de la présente invention, différent des objectifs précédents, est de proposer un procédé de détection et de quantification de composés volatils dans une atmosphère à tester qui propose des résultats rapides.

Un autre objectif de la présente invention, différent des objectifs précédents, est de proposer un élément réflecteur sensible présentant une chauffe optimisée sans perturber la transduction optique.

Afin d'atteindre au moins partiellement, au moins un des objectifs précités, la présente invention a pour objet un dispositif optique de détection de composés volatils, comportant :

- un élément réflecteur sensible dont l'intensité lumineuse réfléchie varie en fonction des composés volatils contenus dans une atmosphère à tester et de leur concentration, l'élément réflecteur sensible comportant :

    * une couche de substrat,
    * au moins une couche sensible transparente entre 400 nm et 1000 nm et poreuse configurée pour permettre l'adsorption et la désorption des composés volatils contenus dans l'atmosphère à tester, et
    * une couche conductrice d'électricité prise en sandwich entre la couche de substrat et la couche sensible et disposée directement au contact de la couche de substrat et de la couche sensible, ladite couche conductrice d'électricité étant configurée pour permettre la chauffe de la couche sensible par effet Joule afin de permettre la désorption des composés volatils adsorbés sur la couche sensible,

- au moins une source de lumière monochromatique ou quasi-monochromatique disposée pour éclairer la couche sensible sous un angle incident,
- au moins un détecteur de lumière configuré pour mesurer l'intensité lumineuse réfléchie par l'élément réflecteur sensible sous un angle de détection, la couche conductrice d'électricité étant transparente et non diffusante entre 400 nm et 1000 nm de façon à ne pas nuire à l'intensité lumineuse réfléchie par l'élément réflecteur sous l'angle de détection, et
- une unité de traitement et de calcul configurée pour déterminer les composés volatils présents dans l'atmosphère à tester selon leurs températures de désorption et leur concentration par variation de l'intensité lumineuse réfléchie par l'élément réflecteur sensible.

Un tel dispositif optique de détection est aisé à mettre en oeuvre car il utilise uniquement une détection de la variation de l'indice de réfraction de l'élément réflecteur sensible afin d'identifier la désorption d'un composé volatil de la couche sensible. De plus, la désorption par voie thermique permet de déterminer aisément et rapidement la nature chimique du composé volatil car chaque composé volatil présente une température de désorption propre selon la composition chimique de la couche sensible. De plus, la présence de l'unité de traitement et de calcul permet de déterminer notamment la concentration en composé volatil désorbé dans l'atmosphère à tester. Ainsi, un tel dispositif optique de détection permet une détection simple et rapide de la présence de composés volatils dans une atmosphère à tester ainsi que la détermination de leur concentration, même à des concentrations faibles, c'est-à-dire de l'ordre du ppm ou encore du ppb.

Le dispositif optique de détection selon la présente invention peut comporter en outre une ou plusieurs des caractéristiques suivantes prises seules ou en combinaison.

Selon un aspect, la présence d'un ou de plusieurs composés volatils désorbé peut être détectée par comparaison de l'intensité lumineuse réfléchie par l'élément réflecteur sensible sans composé volatil adsorbé à une température donnée avec l'intensité lumineuse réfléchie par l'élément réflecteur sensible juste après désorption à cette même température.

La concentration du ou des composés volatils détectés dans l'atmosphère à tester peut être déterminée par intégration de la surface de pics de variation de l'intensité lumineuse réfléchie correspondant à la désorption du ou des composés volatils.

L'au moins une couche sensible peut présenter une épaisseur comprise entre 50 nm et 2000 nm, notamment comprise entre 400 nm et 1200 nm, et plus particulièrement entre 500 nm et 800 nm.

L'au moins une couche sensible peut présenter une taille moyenne de pores inférieure à 2 nm.

Selon un mode de réalisation particulier, l'au moins une couche sensible peut présenter une porosité inférieure à 25 %.

Selon une variante, l'au moins une couche sensible peut être réalisée en silice sol-gel.

Selon une autre variante, l'au moins une couche sensible peut être réalisée par un xérogel.

Selon un aspect de cette autre variante, le xérogel peut comprendre de l'orthosilicate de tétraéthyle (TEOS).

Selon un autre aspect de cette autre variante, le xérogel peut comprendre du triéthoxyméthylsilane (MTEOS).

Selon encore un autre aspect de cette autre variante, le xérogel peut comprendre du phényl-triéthoxysilane (Ph-TEOS).

Selon un mode de réalisation particulier, le xérogel peut comprendre un mélange d'orthosilicate de tétraéthyle (TEOS), de triéthoxyméthylsilane (MTEOS) et de phényl-triéthoxysilane (Ph-TEOS).

Selon un aspect, l'au moins une couche sensible peut présenter un indice de réfraction compris entre 1,2 et 1,6, notamment entre 1,3 et 1,5, pour les longueurs d'ondes comprises entre 400 nm et 1000 nm avant son exposition à l'atmosphère à tester.

De manière alternative ou en complément, l'au moins une couche sensible peut présenter une structuration configurée pour augmenter les variations du signal optique.

La structuration de l'au moins une couche sensible peut comprendre une grille de diffraction.

La structuration de l'au moins une couche sensible peut comprendre des zones de résonance.

La structuration de l'au moins une couche sensible peut comprendre un cristal photonique.

La structuration de l'au moins une couche sensible peut comprendre un empilement de couches.

Selon un premier mode de réalisation, l'élément réflecteur sensible peut comporter une unique couche sensible.

Selon ce premier mode de réalisation, le dispositif optique de détection présente une unique source de lumière et un unique détecteur de lumière.

Selon un deuxième mode de réalisation, l'élément réflecteur sensible peut comporter plusieurs couches sensibles.

Selon ce deuxième mode de réalisation, le dispositif optique de détection présente autant de sources de lumière que de couches sensibles.

Selon ce deuxième mode de réalisation, le dispositif optique de détection peut présenter un unique détecteur de lumière lorsque les longueurs d'onde d'émission des sources de lumière sont différentes.

De manière alternative, le dispositif optique de détection peut présenter autant de détecteurs de lumière que de sources de lumière.

Selon ce deuxième mode de réalisation, les couches sensibles de l'élément réflecteur sensible peuvent présenter des affinités chimiques perpendiculaires afin de permettre une identification chimique du composé chimique plus précise.

Selon une première variante, les couches sensibles présentant des affinités chimiques perpendiculaires peuvent être disposées côte-à-côte.

Selon une deuxième variante, les couches sensibles présentant des affinités chimiques perpendiculaires peuvent être séparées l'une de l'autre dans l'élément réflecteur sensible.

Selon un aspect, la couche de substrat peut être réalisée en un matériau semi-conducteur, notamment en silicium, en verre, en saphir, ou en métal.

Selon un mode de réalisation particulier, la couche de substrat peut posséder un indice de réfraction supérieur à 2,5, notamment supérieur à 3, pour les longueur d'ondes comprises entre 250 nm et 1500 nm.

L'angle incident et l'angle de détection peuvent être respectivement compris entre 30° et 75° par rapport à une normale de le couche sensible.

Selon un mode de réalisation particulier, la longueur d'onde émise par la source de lumière est comprise entre 400 nm et 1000 nm.

Selon un aspect de ce mode de réalisation particulier, la longueur d'onde émise par la source de lumière peut être monochromatique et choisie avec l'angle d'incidence de façon à coïncider avec la position en longueur d'onde d'une inflexion entre deux pics d'interférence constructive et destructive du spectre de réflexion de l'élément réflecteur sensible.

La couche conductrice d'électricité peut présenter une épaisseur inférieure ou égale à 150 nm, notamment comprise entre 70 nm et 90 nm.

Selon un aspect, la couche conductrice d'électricité peut présenter une résistivité comprise entre $10^{-4}$ $\Omega$.m et $10^{-7}$ $\Omega$.m de manière à permettre la chauffe de la couche sensible par effet Joule.

Selon un aspect, la résistance du matériau constitutif de la couche conductrice d'électricité est comprise entre 2 $\Omega$ et 200 $\Omega$, et notamment comprise entre 20 $\Omega$ et 50 $\Omega$.

La couche conductrice d'électricité peut être réalisée en oxyde conducteur transparent, notamment en oxyde d'indium dopé à l'étain (ITO), en oxyde de zinc dopé à l'aluminium (AZO), en oxyde de zinc dopé au gallium (GZO), ou en oxyde d'étain dopé au fluor (FTO).

Selon un mode de réalisation particulier, le dispositif de détection optique peut présenter une enceinte dans laquelle est disposé l'élément réflecteur sensible.

L'enceinte peut présenter au moins une ouverture configurée pour permettre l'entrée et/ou la sortie de l'atmosphère à tester.

La présente invention a également pour objet un procédé de détection et de quantification de composés volatils dans une atmosphère à tester mettant en oeuvre un dispositif de détection optique tel que décrit précédemment. Le procédé de détection et de quantification comprend les étapes suivantes :

- éclairage de la couche sensible sous un angle incident avec la source de lumière monochromatique ou quasi-monochromatique et mesure de l'intensité lumineuse réfléchie par l'élément réflecteur sensible,

- exposition de l'élément réflecteur sensible à l'atmosphère à tester pendant une durée prédéterminée de manière à permettre l'adsorption de composés volatils contenus dans cette atmosphère à tester sur la couche sensible de l'élément réflecteur sensible,

- chauffe de la couche sensible par la couche conductrice d'électricité afin de permettre une désorption contrôlée des composés volatils de la couche sensible,
- mesure de l'intensité lumineuse réfléchie par l'élément réflecteur sensible au cours de l'étape de chauffe afin de déterminer une variation de l'indice de réfraction de l'élément réflecteur sensible, et
- suivi de l'évolution de l'indice de réfraction de l'élément réflecteur sensible afin de déterminer la nature et la quantité de composé volatil désorbé de la couche sensible lors de l'étape de chauffe, ladite étape de suivi de l'évolution de l'indice de réfraction de l'élément réflecteur sensible étant réalisée par l'unité de traitement et de calcul.

Le procédé de détection et de quantification selon la présente invention permet entre autre une adsorption des composés volatils sur la couche sensible à température ambiante. Ensuite, la détection et la quantification des composés volatils adsorbés est réalisée par simple détection d'une variation de l'indice de réfraction de l'élément réflecteur sensible, ce qui permet notamment d'obtenir une réponse rapide concernant l'identification du composé volatil détecté ou encore la une concentration de ce composé volatil dans l'atmosphère à tester.

Le procédé de détection et de quantification de composés volatils dans une atmosphère à tester selon la présente invention peut comporter en outre une ou plusieurs des caractéristiques suivantes prises seules ou en combinaison.

Selon une alternative, les étapes d'éclairage de la couche sensible et d'exposition de l'élément réflecteur sensible à l'atmosphère à tester peuvent être interchangées.

Selon un aspect, l'étape de chauffe de la couche sensible peut être réalisée par une chauffe contrôlée suivant une loi de chauffe comprise entre 1°C/s et 20°C/s.

Selon un mode de réalisation particulier, la détermination de la nature du composé volatil désorbé au cours de l'étape de chauffe est réalisée par comparaison de l'indice de réfraction de l'élément réflecteur sensible à une température donnée par rapport à l'indice de réfraction de cet élément réflecteur sensible sans composé adsorbé à cette même température.

Selon une alternative, la détermination de la nature du composé volatil désorbé est réalisée en traçant la courbe de variation d'indice de réfraction de l'élément réflecteur sensible en fonction de la température de la couche conductrice d'électricité et en déterminant la température de désorption qui correspond à la température pour laquelle la valeur de la dérivée de cette courbe est nulle, par résolution de l'équation :

$$[\text{Math. 1}]$$

$$dN1/dT = 0$$

avec :

- T : la température de la couche conductrice d'électricité en °C,
- N1 : la valeur de l'indice de réfraction de l'élément réflecteur sensible.

Selon une autre alternative, la détermination de la quantité de composé volatil désorbé est réalisée en comparant la variation d'indice de réfraction avant et après chauffage.

Selon cette autre alternative, la quantité de composé volatil désorbé est proportionnelle à l'équation : N2-N1, avec :

- N1 : la valeur de l'indice de réfraction de l'élément réflecteur sensible avec le composé volatil adsorbé
- N2 : la valeur de l'indice de réfraction de l'élément réflecteur sensible sans le composé volatil.

Selon un aspect, l'étape de mesure de l'intensité lumineuse réfléchie par la couche sensible peut être réalisée selon un intervalle de temps compris entre 0.2 seconde et 5 secondes.

La quantification de composés volatils désorbé peut être réalisée par intégration d'une surface d'un pic de variation de l'indice de réfraction de l'élément réflecteur sensible correspondant à la désorption du composé volatil correspondant.

Selon une variante, l'identification du composé volatil, dans le cas où il y a mélange de composés volatils, peut être réalisée par déconvolution de l'indice de réfraction de l'élément réflecteur sensible lors de la désorption de ce composé volatil.

Selon un mode de réalisation particulier, la longueur d'onde de la source lumineuse peut être choisie de façon à coïncider avec la position en longueur d'onde d'une inflexion entre deux pics d'interférence constructive et destructive du spectre de réflexion de l'élément réflecteur sensible.

D'autres caractéristiques et avantages de la présente invention apparaîtront plus clairement à la lecture de la description suivante, donnée à titre illustratif et non limitatif, et des dessins annexés dans lesquels :

[Fig.1] la figure 1 est une représentation schématique en perspective d'un dispositif optique de détection ;

[Fig. 2] la figure 2 est une représentation schématique en coupe transversale d'un élément réflecteur sensible du dispositif optique de détection de la figure 1.

[Fig.3A] la figure 3A est une représentation schématique en coupe transversale d'un élément réflecteur sensible du dispositif optique de détection de la figure 1 selon un premier mode de réalisation ;

[Fig.3B] la figure 3B est une représentation schématique en coupe transversale d'un élément réflecteur sensible du dispositif optique de détection de la figure 1 selon un deuxième mode de réalisation ;

[Fig. 4A] le figure 4A est une représentation schématique en coupe transversale du dispositif optique de détection de la figure 1 selon une première variante ;

[Fig. 4B] la figure 4B est une représentation schématique en coupe transversale du dispositif optique de détection de la figure 1 selon une deuxième variante ;

[Fig. 4C] le figure 4C est une représentation schématique en coupe transversale du dispositif optique de détection de la figure 1 selon une troisième variante ;

[Fig.5] la figure 5 est une représentation schématique d'un organigramme illustrant différentes étapes d'un procédé de détection et de quantification de composés volatils dans une atmosphère à tester mettant en oeuvre le dispositif optique de détection de la figure 1 ;

[Fig.6A] la figure 6A est une représentation schématique d'une couche sensible du dispositif optique de détection de la figure 1 au cours du procédé de détection et de quantification de composés volatils ;

[Fig. 6B] la figure 6B est une représentation schématique de courbes obtenues après traitement au cours d'une désorption de composés volatils de la couche sensible de la figure 6A;

[Fig. 7A-7F] les figures 7A à 7F sont des représentations schématiques de courbes de variation d'indice de réfraction obtenues au cours de désorptions de différents composés volatils isolés pour une couche sensible présentant une composition chimique particulière ; et

[Fig. 8A-8C] les figures 8A à 8C sont des représentations schématiques de courbes de variation d'indice de réfraction obtenues au cours d'une désorption de composés volatils contenus dans une même atmosphère à tester pour des couches sensibles présentant des compositions chimiques différentes.

Les éléments identiques sur les différentes figures, portent les mêmes références.

Les réalisations suivantes sont des exemples. Bien que la description se réfère à un ou plusieurs modes de réalisation, ceci ne signifie pas nécessairement que chaque référence concerne le même mode de réalisation, ou que les caractéristiques s'appliquent seulement à un seul mode de réalisation. De simples caractéristiques de différents modes de réalisation peuvent également être combinées et/ou interchangées pour fournir d'autres réalisations.

Dans la présente description, on peut indexer certains éléments ou paramètres, comme par exemple premier élément ou deuxième élément ainsi que premier paramètre et second paramètre ou encore premier critère et deuxième critère etc. Dans ce cas, il s'agit d'un simple indexage pour différencier et dénommer des éléments, paramètres, ou critères proches mais non identiques. Cette indexation n'implique pas une priorité d'un élément, paramètre ou critère par rapport à un autre et on peut aisément interchanger de telles dénominations sans sortir du cadre de la présente description. Cette indexation n'implique pas non plus un ordre dans le temps par exemple pour apprécier tels ou tels critères.

Dans la présente description, on entend par « composé volatil » un composé qui se vaporise ou s'évapore facilement aux conditions standard de température et de pression, c'est-à-dire 25°C, 1 bar, telles que définies par l'Union Internationale de la Chimie Pure et Appliquée. Dans la présente description, un composé volatil peut être de nature organique, il peut par exemple s'agir d'un composé organique volatil (également connu sous l'acronyme COV), ou encore de nature inorganique, comme l'eau par exemple.

De plus, dans la description suivante, on entend par « transparent » un matériau de préférence incolore, à travers lequel la lumière peut passer avec une absorption d'intensité maximum de 10% pour les longueurs d'ondes comprises en particulier entre 280 nm et 1300 nm.

D'autre part, dans la description suivante, on entend par « xérogel », un matériau à réseau macromoléculaire d'oxydes, vitreux, fabriqué par un procédé sol-gel.

Ensuite, on entend dans la description suivante par « quasi-monochromatique », une onde dont le spectre n'occupe qu'une bande très étroite en longueur d'onde, le spectre de cette onde peut par exemple occuper une bande de longueur d'onde inférieure à 2 nm.

Dispositif optique de détection :

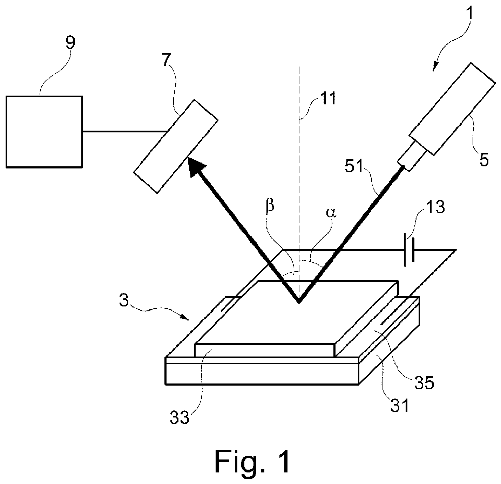

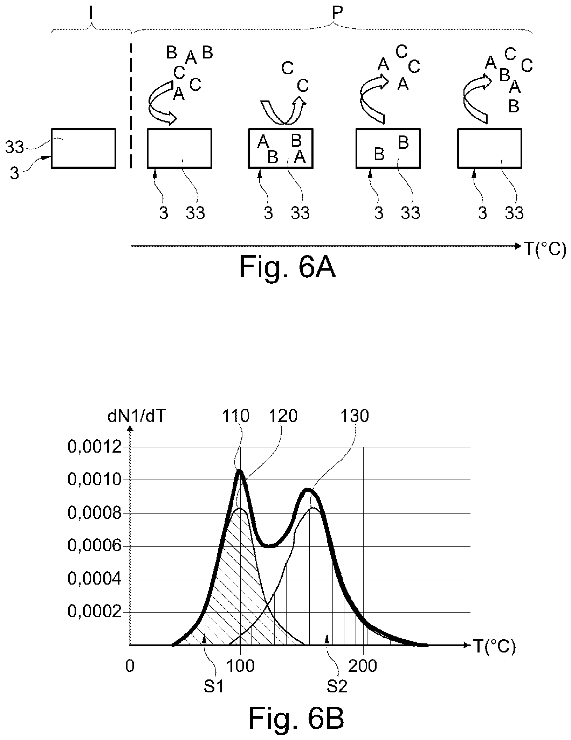

En référence aux figures 1 à 4C, il est représenté un dispositif optique de détection 1 de composés volatils A, B, C (visibles sur la figure 6A). Le dispositif optique de détection 1 comporte un élément réflecteur sensible 3, au moins une source de lumière 5 monochromatique ou quasi-monochromatique, au moins un détecteur de lumière 7, et une unité de traitement et de calcul 9.

L'élément réflecteur sensible 3 est destiné à être placé dans une atmosphère à tester de manière à ce qu'au moins une partie des composés volatils présents dans cette atmosphère à tester sont adsorbés par cet élément réflecteur sensible 3 afin de pouvoir détecter leur présence et déterminer leur concentration lors de leur désorption. Ainsi, l'intensité de réflexion de l'élément réflecteur sensible 3 est destinée à varier en fonction des composés volatils A, B, C contenus dans l'atmosphère à tester adsorbés par cet élément réflecteur sensible 3 et de leur concentration. L'élément réflecteur sensible 3 comporte une couche de substrat 31, au moins une couche sensible 33, et une couche conductrice d'électricité 35 prise en sandwich entre la couche de substrat 31 et la couche sensible 33. D'autre part, la couche conductrice d'électricité 35 est configurée pour chauffer la couche sensible 33 par effet Joule.

La couche de substrat 31 peut être réalisée en un matériau semi-conducteur, notamment en silicium, en verre, en saphir, ou en métal. D'autre part, la couche de substrat 31 possède un indice de réfraction supérieur à 2,5, notamment supérieur à 3, pour les longueur d'ondes comprises entre 250 nm et 1500 nm. De plus, la couche de substrat 31 peut être réfléchissante, par exemple du fait d'un revêtement métallique de type miroir sur une de ses faces disposée en regard de la couche conductrice d'électricité 35.

Par ailleurs, l'au moins une couche sensible 33 est poreuse et transparente. Cette couche sensible 33 est configurée pour permettre l'adsorption et la désorption d'au moins une partie des composés volatils A, B, C (visibles sur la figure 6A) contenus dans l'atmosphère à tester. Cette couche sensible 33 peut présenter une affinité chimique particulière avec certains composés volatils, comme par exemple des dérivés halogénés tels que le chloroforme par exemple, pour les aldéhydes ou les cétones comme par exemple l'acétylacétone, pour les hydrocarbures linéaires saturés ou insaturés ou encore cycliques, comme par exemple l'hexane ou le toluène, les alcools comme par exemple l'éthanol ou l'éthylène glycol. Plus particulièrement, l'adsorption des composés volatils sur cette couche sensible 33 correspond à une physisorption, c'est-à-dire que cette adsorption est réalisée grâce à des forces de faible énergie et en particulier grâce aux forces de Van der Walls. Ainsi, il est possible de favoriser ou non l'adsorption de certaines familles de composés volatils en jouant sur la nature et la composition de cette couche sensible 33. De plus, il est également possible de jouer sur la sélectivité de cette couche sensible 33 à une famille particulière de composés volatils A, B, C en modifiant l'encombrement stériques des pores ou encore leur affinité chimique. L'au moins une couche sensible 33 peut présenter une taille moyenne de pores inférieure à 2 nm et une porosité inférieure à 25 %. De telles propriétés pour la couche sensible 33 confèrent à cette couche sensible 33 des propriétés d'adsorption adaptées pour de nombreux composés volatils A, B, C. En effet, dans le cas où les pores présentent une taille trop importante, il est possible que certains composés se désorbent facilement et cela peut compromettre la précision de mesure de ces composés volatils. On peut déterminer le diamètre moyen des pores par une méthode connue de volumétrie qui est par exemple décrite en détail dans l'article « Porosity and mechanical properties of mesoporous thin films assessed by environmental ellipsometric porosimetry » Cedric Boissière et al, paru dans American Chemical Society, 2005. Langmuir: the ACSjournal ofsurfaces and colloids 2005, 21, 12362-71. De plus, la couche sensible 33 présente une surface accessible de pores supérieure à 140 $cm^2/cm^2$. D'autre part, l'au moins une couche sensible 33 peut présenter un indice de réfraction compris entre 1,2 et 1,6, notamment entre 1,3 et 1,5, pour les longueurs d'ondes comprises entre 400 nm et 1000 nm avant son exposition à l'atmosphère à tester. Des tests menés par les inventeurs montrent qu'une couche sensible 33 présentant de telles propriétés permet d'avoir une sensibilité comprise entre $10^{-4}$ et $10^{-5}$ unité optique par ppm de composé volatil A, B, C.

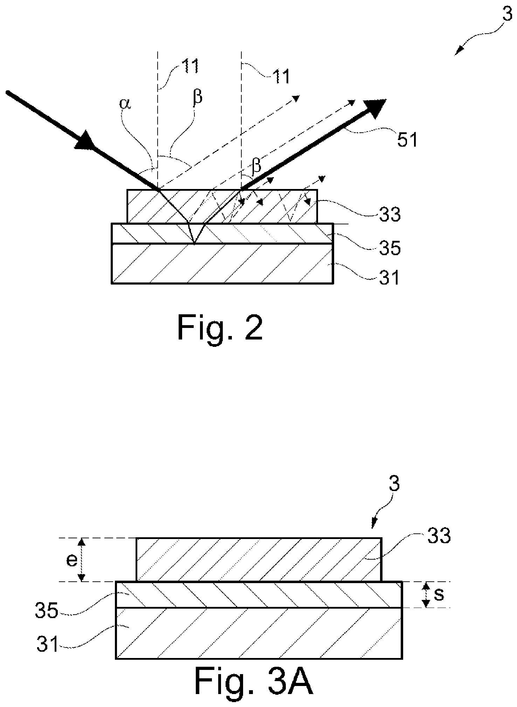

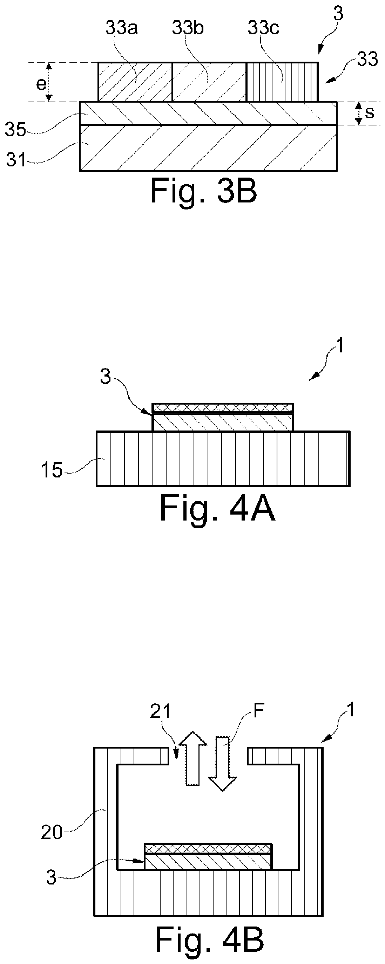

L'au moins une couche sensible 33 peut être réalisée en silice sol-gel, ou encore être un xérogel. Grâce à l'utilisation de procédés sol-gel, il est possible de contrôler aisément la formation de cette couche sensible 33 et notamment son épaisseur e (visible sur les figures 3A et 3B). Plus particulièrement, l'au moins une couche sensible 33 présente une épaisseur e comprise entre 50 nm et 2000 nm, notamment comprise entre 400 nm et 1200 nm, et plus particulièrement entre 500 nm et 800 nm. L'épaisseur e de la couche sensible 33 permet d'avoir des temps de réponse assez courts. En effet, si l'épaisseur e de la couche sensible 33 est trop importante, cela entraîne un temps d'équilibre assez long, ce qui peut être considéré comme gênant par un utilisateur du dispositif optique de détection 1. De plus l'épaisseur e de la couche sensible 33 permet de placer un point d'inflexion de pente maximale à une longueur d'onde de mesure correspondant à la longueur d'onde d'émission de la source de lumière 5. De plus, une telle épaisseur e de la couche sensible 33 permet à celle-ci d'avoir une température uniforme lors de sa chauffe. En effet, si l'épaisseur e de la couche sensible 33 est trop importante, alors la température de cette dernière ne sera pas uniforme sur l'ensemble de son épaisseur e au cours de sa chauffe, ce qui peut nuire aux mesures effectuées par le dispositif optique de détection 1.

Par ailleurs, l'au moins une couche sensible 33 ne comprend pas d'agents structurants, c'est-à-dire d'espèces chimiques de nature minérale ou organique autour desquelles le matériau constitutif de la couche sensible 33 pourrait s'organiser

D'autre part, dans le cas où la couche sensible 33 est formée par un xérogel, ce xérogel peut comprendre de l'orthosilicate de tétraéthyle (TEOS), du triéthoxyméthylsilane (MTEOS), ou encore du phényl-triéthoxysilane (Ph-TEOS). Selon un mode de réalisation particulier développé plus en détail ultérieurement, le xérogel peut comprendre un mélange d'orthosilicate de tétraéthyle (TEOS), de triéthoxyméthylsilane (MTEOS) et de phényl-triéthoxysilane (Ph-TEOS).

Par ailleurs, la couche conductrice d'électricité 35 de l'élément réflecteur sensible 3 est non diffusante, c'est-à-dire qu'elle ne diffuse pas la lumière, et transparente aux longueurs d'ondes comprises entre 400 nm et 1000 nm. En effet, si cette couche conductrice d'électricité 35 diffusait la lumière, cela pourrait éventuellement nuire à la précision de la mesure effectuée. En effet, si la couche conductrice d'électricité 35 est diffusante, une partie du rayon lumineux 51 peut être diffusé, ce qui peut nuire à l'intensité de ce signal une fois réfléchi et rendre ce dernier difficile à analyser par le détecteur de lumière du fait d'un masquage du signal initial lié à la diffusion de ce dernier dans la couche conductrice d'électricité 35. De plus, si cette couche conductrice d'électricité 35 est opaque, dans ce cas, il n'est pas possible d'obtenir de rayon réfléchi et donc de détection de ce dernier par le détecteur de lumière 7. Cette couche conductrice d'électricité 35 est disposée directement au contact de la couche de substrat 31 et de la couche sensible 33. Cette couche conductrice d'électricité 35 est configurée pour permettre la chauffe de la couche sensible 31 par effet Joule afin de permettre la désorption des composés volatils A, B, C adsorbés sur la couche sensible 33. A cet effet, la couche conductrice d'électricité 35 peut présenter une résistivité comprise entre $10^{-4}$ $\Omega$.m et $10^{-7}$ $\Omega$.m. D'autre part, le composant de cette couche conductrice d'électricité 35 peut présenter une résistance comprise entre 2 $\Omega$ et 200 $\Omega$, et notamment comprise entre 20 $\Omega$ et 50 $\Omega$, de manière à permettre la chauffe de la couche sensible 33 par effet Joule. La disposition de cette couche conductrice d'électricité 35 directement au contact de la couche sensible 33 permet une chauffe efficace de cette dernière et limite les temps nécessaires à la désorption des composés volatils A, B, C adsorbés sur la couche sensible 33. De plus, la couche conductrice d'électricité 35 peut présenter une épaisseur s (visible sur les figures 3A et 3B) inférieure ou égale à 150 nm, notamment comprise entre 70 nm et 90 nm. L'épaisseur s de cette couche conductrice d'électricité 35 peut être ajustée de façon à ne pas perturber la lecture optique et également de façon à ajuster la résistivité de cette couche conductrice d'électricité 35 afin d'adapter la puissance électrique nécessaire aux conditions de chauffage. D'autre part, la couche conductrice d'électricité 35 peut par exemple être réalisée en oxyde conducteur transparent (TCO), notamment en oxyde d'indium dopé à l'étain (ITO), en oxyde de zinc dopé à l'aluminium (AZO), en oxyde de zinc dopé au gallium (GZO), ou en oxyde d'étain dopé au fluor (FTO). De tels composés présentent des propriétés de résistivité compatibles avec celles requises afin de permettre la chauffe de la couche sensible 33 par effet Joule. Une telle composition, en oxyde conducteur transparent, de la couche conductrice d'électricité 35 permet une transduction optique par réflexion directe d'un faisceau lumineux 51 émis par la source de lumière 5. D'autre part, les oxydes conducteurs transparents (TCO) sont de bons candidats pour une élaboration sol-gel, et donc pour la formation de la couche sensible 33. Selon ce mode de réalisation particulier, la couche de substrat 31 présente un revêtement métallique de type miroir sur sa face disposée au contact de la couche conductrice d'électricité 35 afin d'améliorer l'intensité du rayon lumineux réfléchi.

Ainsi, l'élément réflecteur sensible 3 présente une épaisseur faible ce qui permet de faciliter sa disposition aux endroits de mesure ou encore son transport. De plus, l'élément réflecteur sensible 3 peut être disposé aux endroits de mesure sans nécessiter la présence des autres éléments du dispositif optique de détection 1, ceux-ci n'étant nécessaires que lors de l'étude de la désorption des composés volatils A, B, C éventuellement adsorbés sur la couche sensible 33.

Par ailleurs, la source de lumière 5 est disposée pour éclairer la couche sensible 33 sous un angle incident $\alpha$. Selon le mode de réalisation particulier de la figure 1, la longueur d'onde émise par la source de lumière 5 est comprise entre 400 nm et 1000 nm. Par ailleurs, la longueur d'onde émise par la source de lumière 5 peut être monochromatique et choisie avec l'angle d'incidence $\alpha$ de façon à coïncider avec la position en longueur d'onde d'une inflexion entre deux pics d'interférence constructive et destructive du spectre de réflexion de l'élément réflecteur sensible 3. L'épaisseur e de la couche sensible 33 est choisie suffisamment grande pour exalter les pentes des zones d'inflexion du spectre de réflexion sans que le nombre d'interférences ne dépasse une valeur qui rendrait l'alignement trop délicat.

De plus, le détecteur de lumière 7 est configuré pour mesurer l'intensité lumineuse réfléchie par l'élément réflecteur sensible 3 sous un angle de détection $\beta$. Ce détecteur de lumière 7 peut par exemple être une caméra ou tout autre élément apte à détecter la rayon lumineux 51 réfléchi par l'élément réflecteur sensible 3. Selon le mode de réalisation particulier de la figure 1, l'angle incident $\alpha$ et l'angle de détection $\beta$ sont respectivement compris entre 30° et 75° par rapport à une normale 11 de le couche sensible 33. Cette normale 11 est une ligne imaginaire disposée perpendiculairement à la couche sensible 33. D'autre part, les angles incident α et de détection β peuvent être égaux dans le cas d'une réflexion directe, mais ce n'est pas nécessaire. En référence à la figure 2, il est représenté le chemin optique du rayon lumineux 51 dans la couche sensible 31 et dans la couche conductrice d'électricité 35. Comme représenté sur cette figure 2, les angles incident α et de réflexion β sont déterminés par rapport à la normale 11 de la couche sensible 33. Sur cette figure 2, la représentation du chemin optique a été volontairement exagérée pour une bonne identification des angles incident α et de détection β ainsi que les différentes déviations de ce rayon lumineux 51 qui se produisent aux interfaces des couches sensible 33 et conductrice d'électricité 35.

Selon une approche simplifiée avec les équations de Fresnel ne prenant pas en compte la présence de la couche de substrat 31, on peut poser :

[Math. 2]

$$Rs = \left| \frac{n_1 \cos(\alpha) - n_2 \cos(\beta)}{n_1 \cos(\alpha) + n_2 \cos(\beta)} \right|^2$$

[Math. 3]

$$Rp = \left| \frac{n_1 \cos(\beta) - n_2 \cos(\alpha)}{n_1 \cos(\beta) + n_2 \cos(\alpha)} \right|^2$$

[Math. 4]

$$R = 1/2 (Rs + Rp)$$

où

- Rs représente la réflectivité pour polarisation incidente s,
- Rp représente la réflectivité pour polarisation incidente p,
- R représente la réflectivité dans le cas de lumière non-polarisée,
- α représente l'angle du rayon lumineux 51 incident,
- β représente l'angle du rayon lumineux 51 réfléchi,
- $n_1$ est l'indice de réfraction de l'air, $n_1 = 1$ = constant,
- $n_2$ est l'indice de réfraction de l'élément réflecteur sensible 3 qui varie en fonction de l'adsorption de composés volatils A, B, C contenus dans l'atmosphère à tester par la couche sensible 33.

Etant donné que dans cette formule, seul $n_2$ varie en fonction de l'adsorption de composés volatils A, B, C contenus dans l'atmosphère à tester par la couche sensible 33, $n_1$, α, β restant constants, on peut donc en déduire que le taux de réflexion diminue linéairement avec une augmentation de l'indice de réfraction $n_2$. Il se trouve que l'indice de réfraction $n_2$ de l'élément réflecteur sensible 3 varie linéairement avec la quantité de composés volatils A, B, C adsorbée par la couche sensible 33.

En revenant sur la figure 1, l'unité de traitement et de calcul 9 est configurée pour déterminer les composés volatils A, B, C présents dans l'atmosphère à tester selon leurs températures de désorption et leur concentration par variation de l'intensité lumineuse réfléchie par l'élément réflecteur sensible 3. L'unité de traitement et de calcul 9 permet de détecter la présence d'un ou de plusieurs composés volatils A, B, C désorbé de la couche sensible 33 par comparaison de l'intensité lumineuse réfléchie par l'élément réflecteur sensible 3 à une température donnée avec l'intensité lumineuse réfléchie par cet élément réflecteur sensible 3 avant son exposition à l'atmosphère à tester à cette même température comme cela est décrit plus en détail ultérieurement. De plus, la concentration du ou des composés volatils A, B, C détectés dans l'atmosphère à tester est déterminée par intégration de la surface de chaque pic de variation de l'indice de réfraction de l'élément réflecteur sensible 3, correspondant à la désorption de composés volatils A, B, C. Cette intégration peut notamment être réalisée après calibration de l'unité de traitement et de calcul 9.

Ainsi, le dispositif optique de détection 1 permet de détecter aisément la présence d'un ou de plusieurs composés volatils A, B, C dans une atmosphère à tester par simple variation de l'intensité de réflexion d'un rayon lumineux 51 permettant de déterminer une variation de l'indice de réfraction de l'élément réflecteur sensible 3. Cette détection peut donc être rapide. En effet, la transduction étant de type réflexion optique, la détection de composés volatils A, B, C se fait par mesure de la variation de l'intensité réfléchie à une seule longueur d'onde qui est induite par l'augmentation de l'indice de réfraction associé à l'adsorption du composé volatil A, B, C dans la couche sensible 33. De plus, l'utilisation d'une désorption thermique est simple et rapide à réaliser afin de détecter et de quantifier le composé volatil A, B, C se désorbant et initialement présent dans l'atmosphère à tester. De plus, les différents composants de l'élément réflecteur sensible 3 sont assez bon marché, ce qui permet de limiter les coûts de fabrication de ce dispositif optique de détection 1.

Par ailleurs, la sensibilité du dispositif optique de détection 1 peut être exaltée en choisissant judicieusement en particulier l'épaisseur e de la couche sensible 33 d'une part et la longueur d'onde combinée avec l'angle d'incidence $\alpha$ d'autre part. En effet, ci-dessus des équations de Fresnel simplifiées ont été présentées pour expliquer la base de fonctionnement du dispositif optique de détection 1.

De manière alternative ou en complément, l'au moins une couche sensible 33 peut présenter une structuration configurée pour augmenter les variations du signal optique afin d'améliorer la sensibilité de ce dispositif optique de détection 1. La structuration de l'au moins une couche sensible 33 peut comprendre une grille de diffraction, des zones de résonance, un cristal photonique, ou encore un empilement de couches.

Selon le mode de réalisation particulier de la figure 3A, l'élément réflecteur sensible 3 peut comporter une unique couche sensible 33. Cette couche sensible 33 peut être sélective à une unique famille de composés chimiques ou encore à une pluralité de familles de composés chimiques. Lorsque l'élément réflecteur sensible 3 présente une unique couche sensible 33, le dispositif optique de détection 1 comporte une unique source de lumière 5 et un unique détecteur de lumière 7. Selon ce mode de réalisation particulier, la couche conductrice d'électricité 35 présente une épaisseur s de 80 nm, une surface de 5*5mm$^2$, et une résistance de 30 $\Omega$. Une telle couche conductrice d'électricité 35 lorsqu'elle est alimentée par le générateur 13 (visible sur la figure 1) à une tension de 4,5 V permet à la couche sensible 33 d'atteindre une température de 80°C en 25 secondes ; ou encore lorsque le générateur 13 fournit 12V à cette couche conductrice d'électricité 35, la température de la couche sensible 33 atteint une température de 300°C. La présence d'une unique couche sensible 33 pour l'élément réflecteur sensible 3 permet de faciliter sa fabrication. Toutefois, lorsque des composés volatils A, B, C ont des températures de désorption de cette couche sensible 33 proches, il peut être possible de ne pas arriver à distinguer ces composés volatils A, B, C, comme cela est expliqué ultérieurement. De telles situations sont assez rares mais peuvent se produire.

Afin de palier à cette éventualité, et comme représenté en référence à la figure 3B, l'élément réflecteur sensible 3 peut comporter plusieurs couches sensibles 33 disposées adjacentes les unes des autres sur la couche conductrice d'électricité 35. Afin de permettre une discrimination de composés volatils A, B, C présentant une température de désorption proche, voire identique, ce qui pourrait se produire avec la couche sensible 33 de la figure 3A, les couches sensibles 33a, 33b, 33c de l'élément réflecteur sensible 3 peuvent présenter des affinités chimiques perpendiculaires, permettant ainsi une identification chimique des composés volatils A, B, C plus précise. On entend par affinités chimiques perpendiculaires, le fait qu'une des couches sensible 33a, 33b, 33c présente une affinité chimique élevée, voire exclusive, pour une première famille de composés chimiques et négligeable, voire nulle, pour une deuxième famille de composés chimiques, et qu'une autre des couches sensibles 33a, 33b, 33c présente une affinité chimique élevée, voire exclusive, pour cette deuxième famille de composés chimiques, et négligeable, voire nulle, pour cette première famille de composés chimiques.

Selon la représentation particulière de la figure 3B, l'élément réflecteur sensible 3 comporte trois couches sensibles 33a, 33b, 33c. Les couches sensibles 33a, 33b, 33c présentent des affinités chimiques perpendiculaires correspondent aux couches sensibles 33a et 33b, elles sont donc disposées côte-à-côte. De manière alternative, les couches sensibles 33a, 33b, 33c présentant des affinités chimiques perpendiculaires correspondent aux couches sensibles 33a et 33c, elles sont donc séparées l'une de l'autre dans l'élément réflecteur sensible 3. De manière évidente, les compositions des couches sensibles 33a, 33b, 33c sont différentes les unes des autres et les températures de désorption des composés volatils A, B, C de ces couches sensibles 33a, 33b, 33c peuvent être différentes les unes des autres selon la couche sensible 33a, 33b, 33c considérée comme cela est décrit plus en détail ultérieurement. En effet, en changeant la composition chimique des couches sensibles 33a, 33b, 33c, l'affinité chimique de ces couches sensibles 33a, 33b, 33c avec un même composé volatil A, B, C est différente et la température de désorption d'un même composé volatil A, B, C, de couches sensibles 33a, 33b, 33c présentant des compositions chimiques différentes peut varier. Selon ce mode de réalisation particulier, le dispositif optique de détection 1 comporte autant de sources de lumière 5 que de couches sensibles 33a, 33b, 33c différentes, chaque source de lumière 5 dirigeant un faisceau lumineux 51 en direction d'une couche sensible 33a, 33b, 33c propre. Afin de suivre l'évolution de la propriété optique de la couche sensible 33 en fonction de la température au cours de la désorption des composés volatils A, B, C, le dispositif optique de détection 1 peut présenter un unique détecteur de lumière 7 dans le cas où les différentes sources de lumière 5 émettent des faisceaux lumineux 51 de longueurs d'ondes différentes. De manière alternative, le dispositif optique de détection 1 peut présenter autant de dispositifs de détection 7 que de sources de lumière 5, chaque dispositif de détection 7 étant associé à une source de lumière 5 particulière et à une couche sensible 33a, 33b, 33c particulière. Un élément réflecteur sensible 3 selon le mode de réalisation particulier de la figure 3B peut par exemple constituer un nez artificiel.

En référence aux figures 4A à 4C, il est représenté différentes variantes du dispositif optique de détection 1.

En référence à la figure 4A, l'élément réflecteur sensible 3 peut être disposé sur un socle 15 et laissé à l'air libre afin de permettre l'adsorption de composés volatils A, B, C présents dans l'atmosphère environnant ce dispositif optique de détection 1. Le socle 15 représenté sur la figure 4A peut correspondre à un élément sur lequel l'élément réflecteur sensible 3 est disposé comme par exemple une table. Ce socle 15 peut également correspondre à un adhésif configuré pour permettre la fixation de cet élément réflecteur sensible 3 sur tout type de support. Ainsi, ce socle 15 peut être un élément supplémentaire ajouté à l'élément réflecteur sensible 3 ou être un élément distinct du dispositif optique de détection 1.

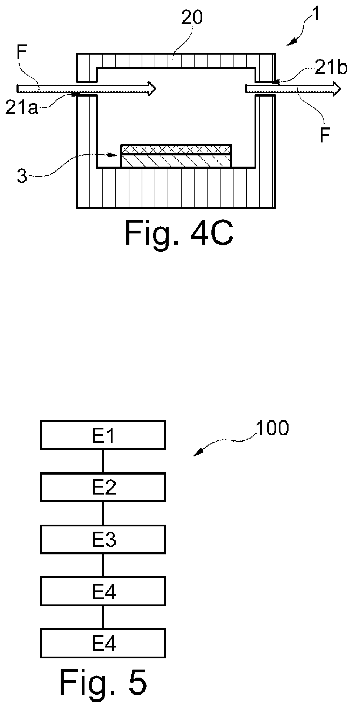

En référence aux figures 4B et 4C, le dispositif de détection optique 1 peut présenter une enceinte 20 dans laquelle est disposé l'élément réflecteur sensible 3. La présence de cette enceinte 20 peut permettre de faciliter les manipulations et les déplacements de l'élément réflecteur sensible 3. En effet, la présence de cette enceinte 20 peut permettre de prévenir d'éventuelles pollutions de la couche sensible 33 par des composés éventuellement présents sur les mains d'un manipulateur. L'enceinte 20 présente au moins une ouverture 21 configurée pour permettre l'entrée et/ou la sortie de l'atmosphère à tester sous la forme d'un flux de gaz F. Plus particulièrement, le dispositif optique de détection 1 de la figure 4B présente une unique ouverture 21 afin de permettre l'entrée et la sortir du flux de gaz F. Un tel dispositif optique de détection 1 peut également être utilisé pour détecter la présence de certains composés volatils dans une atmosphère libre par exemple comme cela peut être le cas pour l'air présent dans une pièce ou dans un habitacle de véhicule automobile. D'autre part, selon le mode de réalisation particulier de la figure 4C, l'enceinte 20 présente une première ouverture 21a configurée pour permettre l'entrée du flux de gaz F dans l'enceinte 20 et une deuxième ouverture 21b configurée pour permettre la sortie de ce flux de gaz F de l'enceinte 20. Selon le mode de réalisation particulier de la figure 4C, le flux d'air F est généré à l'intérieur de l'enceinte 20 à l'aide d'un ventilateur (non représenté) disposé à l'intérieur de cette enceinte 20. Le dispositif optique de détection 1 de la figure 4C peut par exemple être utilisé pour détecter la présence de certains composés volatils présents dans un gaz contenu dans une bouteille par exemple, ou encore dans l'atmosphère d'une pièce ou d'un habitacle de véhicule. Selon les modes de réalisations des figures 4B et 4C, la source de lumière 5 et le détecteur de lumière 7 peuvent être disposés dans cette enceinte 20 de manière à obtenir un dispositif optique de détection 1 portatif. Dans une telle configuration, l'indice de réfraction de la couche sensible 33 peut être mesuré et suivi in situ au cours du chauffage de cette couche sensible 33 par la couche conductrice d'électricité 35. De manière alternative, l'enceinte 20 peut ne contenir que l'élément réflecteur sensible 3, les autres éléments étant disposés à l'extérieur de l'enceinte 20. Selon encore une autre alternative, l'élément réflecteur sensible 3 peut être amovible de cette enceinte 20 afin de permettre la désorption et donc la détection et la quantification des composés volatils A, B, C adsorbés sur l'élément réflecteur sensible 3.

Les inventeurs ont observé qu'en fonction de la présence et de la configuration de l'enceinte 20, les vitesses d'adsorption des composés volatils A, B, C ne sont pas identiques. Plus particulièrement, la configuration de l'enceinte 20 présentant les première 21a et deuxième 21b ouvertures, telle que représentée en référence à la figure 4C, permet une adsorption plus rapide des composés volatils sur la couche sensible 33 et les configurations représentées en référence aux figures 4A et 4B offrent une vitesse d'adsorption sensiblement égales et plus faibles que pour la configuration de l'enceinte 20 de la figure 4C.

Procédé de détection et de quantification :

En référence à la figure 5, il est représenté un organigramme illustrant un procédé de détection et de quantification 100 de composés volatils A, B, C (visibles sur la figure 6A) dans une atmosphère à tester mettant en oeuvre le dispositif de détection optique 1 décrit précédemment.

Le procédé de détection et de quantification 100 comprend une étape d'éclairage E1 de la couche sensible 33 sous un angle incident $\alpha$ avec la source de lumière 5 (visibles sur la figure 1) monochromatique ou quasi-monochromatique et mesure de l'intensité lumineuse réfléchie par l'élément réflecteur sensible 3. Cette étape d'éclairage E1 permet de déterminer une réflexion initiale du rayon lumineux 51 avant toute adsorption de composés volatils A, B, C sur la couche sensible 33.

Le procédé de détection et de quantification 100 met ensuite en oeuvre une étape d'exposition E2 de l'élément réflecteur sensible 3 à l'atmosphère à tester pendant une durée prédéterminée de manière à permettre l'adsorption de composés volatils A, B, C contenus dans cette atmosphère à tester sur la couche sensible 33 de l'élément réflecteur sensible 3. Au cours de cette étape d'exposition E2, la couche sensible 33 de l'élément réflecteur sensible 3 est également éclairée afin de pouvoir constater l'adsorption éventuelle de composés volatils A, B, C sur cette couche sensible 33. En effet, l'indice de réfraction de l'élément réflecteur sensible 3 augmente ou diminue lors de l'adsorption de composés volatils A, B, C. L'éclairage de la couche sensible 33 lors de cette étape d'exposition E2 permet donc de déterminer si des composés volatils A, B, C sont adsorbés par la couche sensible 33.

Selon une alternative, les étapes d'éclairage E1 de la couche sensible 33 et d'exposition E2 de l'élément réflecteur sensible 3 à l'atmosphère à tester peuvent être interverties. Plus particulièrement, l'étape d'exposition E2 de l'élément réflecteur sensible 3 à l'atmosphère à tester peut être mise en oeuvre préalablement à l'étape d'éclairage E1 de la couche sensible 33.

Le procédé de détection et de quantification 100 met ensuite en oeuvre une étape de chauffe E3 de la couche sensible 33 par la couche conductrice d'électricité 35 afin de permettre une désorption des composés volatils A, B, C de la couche sensible 33. Selon un mode de réalisation particulier, l'étape de chauffe E3 de la couche sensible 33 est réalisée par une chauffe contrôlée suivant une loi de chauffe comprise entre 1°C/s et 20°C/s.

Le procédé de détection et de quantification 100 comprend une étape de mesure de l'intensité lumineuse réfléchie E4 par l'élément réflecteur sensible 3 au cours de l'étape de chauffe E3 afin de déterminer une variation de l'indice de réfraction de cet élément réflecteur sensible 3. L'étape de mesure de l'intensité lumineuse réfléchie E4 par l'élément réflecteur sensible 3 peut par exemple être réalisée selon des intervalles de temps compris entre 0,2 seconde et 5 secondes. En effet, la modification de l'indice de réfraction de l'élément réflecteur sensible 3 permet de détecter l'adsorption de composés volatils A, B, C par la couche sensible 33 de ce dernier ou encore la désorption de composés volatils A, B, C de la couche sensible 33 de cet élément réflecteur sensible 3.

Enfin, le procédé de détection et de quantification 100 comprend une étape de suivi de l'évolution E5 de l'indice de réfraction de l'élément réflecteur sensible 3 afin de déterminer la nature chimique et la quantité de composé volatil A, B, C désorbé de la couche sensible 33 lors de l'étape de chauffe E3. Cette étape de suivi de l'évolution E5 de l'indice de réfraction de l'élément réflecteur sensible 3 est réalisée par l'unité de traitement et de calcul 9. Plus particulièrement, au cours de cette étape de suivi de l'évolution E5 de l'indice de réfraction de l'élément réflecteur sensible 3, l'unité de traitement et de calcul détermine la nature du composé volatil A, B, C désorbé au cours de l'étape de chauffe E3 en effectuant la dérivée du signal en fonction de la température et en comparant la valeur de la température pour laquelle la dérivée en question est nulle aux températures présentes dans une base de données. D'autre part, la quantification de composé volatil A, B, C désorbé est réalisée par intégration de la surface des différents pics de variation de l'intensité lumineuse réfléchie correspondant à la désorption du composé volatil désorbé. Cette intégration peut être réalisée après calibration de l'unité de traitement et de calcul 9.

Les inventeurs ont déterminé au cours de différents tests que l'ensemble des composés volatils A, B, C potentiellement adsorbés sur les couches sensibles 33 sont totalement désorbés lorsque cette couche sensible 33 atteint une température supérieure ou égale à 250°C. En suivant une loi de chauffe comprise entre 1°C/s et 20°C/s, on observe que ce procédé de détection et de quantification 100 offre des temps de réponse courts.

En référence à la figure 6A, il est représenté de manière schématique une cinématique du procédé de détection et de quantification 100 décrit en référence à la figure 5. Selon cette cinématique, il est représenté la couche sensible 33 de l'élément réflecteur sensible 3 selon un état initial I et selon un état pollué P au cours du procédé de détection et de quantification 100. Lorsque la couche sensible 33 se trouve dans son état initial I, aucun composé volatil A, B, C n'est adsorbé sur cette dernière. Afin de permettre un étalonnage des mesures, il est possible de réaliser des mesures de l'indice de réfraction de cette couche sensible 33 sans élément adsorbé par celle-ci en fonction de sa température en réalisant une chauffe de cette couche sensible 33 par activation du générateur 13 (visible sur la figure 1) afin qu'il fournisse de l'énergie à la couche conductrice d'électricité 35 pour la chauffe de la couche sensible 33 par effet Joule.

La couche sensible 33 est ensuite exposée à l'atmosphère à tester comprenant éventuellement des composés volatils A, B, C dont au moins une partie est destiné à s'adsorber sur la couche sensible 33. Cette exposition de la couche sensible 33 à l'atmosphère à tester se fait typiquement à température ambiante, c'est à dire 25°C environ. Cette exposition est réalisée pendant une durée prédéterminée afin de permettre l'adsorption d'au moins une partie des composés volatils A, B, C sur cette couche sensible 33. Cette durée d'exposition peut être comprise entre quelques minutes, typiquement 10 à 30min, et plusieurs jours, typiquement 5 ou 6 jours. Selon l'exemple représenté sur la figure 6A, la couche sensible 33 est spécifique aux composés volatils A et B qui peuvent s'adsorber sur cette dernière alors que le composé volatil C présente une affinité chimique négligeable, voire nulle, avec cette couche sensible 33. Le composé volatil C n'est donc pas adsorbé sur celle-ci. La couche sensible 33 est ensuite chauffée par effet Joule lors de l'alimentation de la couche conductrice d'électricité 35 en courant par le générateur 13. Au cours de cette chauffe, les espèces chimiques A et B adsorbées sur cette couche sensible 33 vont se désorber à des températures différentes. Cette désorption différenciée permet de détecter la présence des composés volatils A et B dans cette atmosphère à tester et d'identifier ces composés volatils A et B grâce à leur température de désorption. On constate ainsi que la couche sensible 33 permet d'avoir une double sélectivité par rapport aux composés volatils A, B, C : une première liée à l'adsorption du composé volatil sur cette couche sensible 33, et une deuxième liée à sa température de désorption de cette couche sensible 33. Un tel procédé de mesure et de quantification 100 permet donc d'avoir une sensibilité de mesure élevée.

En effet, comme cela est illustré par la figure 6B, les composés volatils A et B vont se désorber à des températures différentes de cette couche sensible 33. Cette désorption différenciée en fonction de la température de la couche sensible 33 est liée à l'affinité chimique que ces composés chimiques A et B ont avec la couche sensible 33 et à leur chaleur de désorption. En effet, plus la chaleur de désorption et l'affinité chimique entre le composé volatils A, B, C et la couche sensible 33 sont élevées, plus la température de désorption de ce composé volatil A, B, C est élevée. Ainsi, selon la cinématique illustrée sur la figure 6A, le composé volatil B présente une affinité chimique plus élevée avec la couche sensible 33 que le composé volatil A qui va se désorber à une température inférieure, comme illustré sur la figure 6B.

En référence à la figure 6B, la détermination de la nature du composé volatil désorbé est réalisée en traçant la courbe de variation d'indice de réfraction de l'élément réflecteur sensible 3 en fonction de la température de la couche conductrice 35 et en déterminant la température de désorption qui correspond à la température pour laquelle la valeur de la dérivée de cette courbe est nulle par résolution de l'équation :

$$[\text{Math. 1}]$$

$$dN1/dT = 0$$

avec :

- T : la température de la couche conductrice d'électricité 35 en °C,
- N1 : la valeur de l'indice de réfraction de l'élément réflecteur sensible 3.

Plus particulièrement selon la figure 6B, la courbe 110 correspond au tracé de la courbe répondant à cette équation. L'unité de traitement et de calcul 9 (visible sur la figure 1) permet ensuite l'identification du composé volatil A, B, C, dans le cas où il y a mélange de composés volatils A, B, C comme c'est le cas ici. Cette identification du composé volatil A, B, C est réalisée par déconvolution mathématique de la dérivée de l'intensité de lumière réfléchie par l'élément réflecteur sensible 3 en fonction de la température lors de la désorption de ce gaz. Plus particulièrement ces déconvolutions sont illustrées par la courbe 120 représentative du composé volatil A lors de sa désorption et la courbe 130 représentative du composé volatil B lors de sa désorption. Par interprétation des différentes courbes de la figure 6B, on peut déduire que le composé A se désorbe de la couche sensible 33 à une température de 94°C environ et que le composé B désorbe de cette couche sensible 33 à une température de 161°C environ. La détermination de ces température de désorption selon la composition de la couche sensible 33 permet de déterminer la nature du composé volatil A, B, C désorbé. Ces différentes températures de désorption en fonction de la composition de la couche sensible 33 peuvent par exemple être listées dans des tables.

D'autre part, l'intégration d'une surface S1 disposée sous la courbe 120, correspondant à la surface du pic de variation de l'indice de réfraction de l'élément réflecteur sensible 3 liée à la désorption du composé volatil A, permet de déterminer la quantité de composé volatil A adsorbé sur la couche sensible 33 lors de son exposition à l'atmosphère à tester et ainsi de remonter à sa concentration dans cette atmosphère. Typiquement, la surface S1 correspond à la partie présentant des hachures inclinées sous cette courbe 120. D'autre part, la quantification de composé volatil B désorbé est également obtenue par intégration d'une surface S2 disposée sous la courbe 130 et correspondant à la surface du pic de variation de l'indice de réfraction de l'élément réflecteur sensible 3 liée à la désorption du composé volatil B. Cette intégration de la surface S2 permet donc d'obtenir la quantité de composé volatil B désorbé et donc de remonter à sa concentration de l'atmosphère à tester. Cette surface S2 correspond à la partie présentant des hachures verticales sous cette courbe 130. Ainsi, la quantification du composé volatil désorbé est réalisée par traitement mathématique différentiel des courbes d'évolution de la réponse optique en fonction de la température de la couche conductrice d'électricité 35 et donc en fonction de la température de la couche sensible 33.

Selon une alternative non représentée ici, la détermination de la quantité de composé volatil désorbé est réalisée en comparant la variation de l'indice de réfraction avant et après chauffage de la couche sensible 33. Selon cette alternative, la quantité de composé volatil désorbé est proportionnelle à l'équation : N2-N1 avec :

- N1 : la valeur de l'indice de réfraction de l'élément réflecteur sensible 3 avec le composé volatil adsorbé, et
- N2 : la valeur de l'indice de réfraction de l'élément réflecteur sensible sans le composé volatil.

Températures de désorption de certains composés volatils pour une couche sensible donnée :

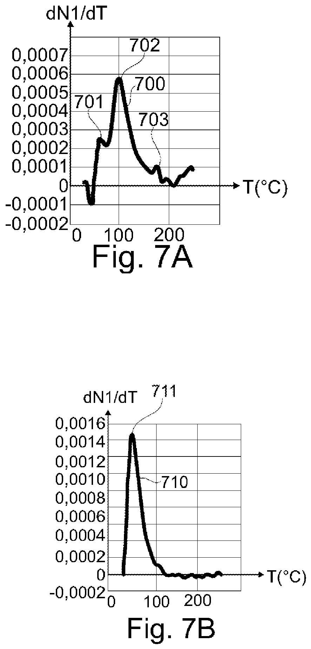

En référence aux figures 7A à 7F, il est représenté différentes courbes illustrant la variation de l'indice de réfraction de l'élément réflecteur sensible 3 en fonction de la température de cette couche sensible 33. Dans ces différents exemples, la couche sensible 33 est de la même composition chimique pour les différentes expériences et le composé volatil mis en présence de cette couche sensible 33 est connu.

La couche sensible 33 utilisée pour démontrer la différence de température de désorption en fonction du composé volatil A, B, C adsorbé présente une composition S3M3P3. Pour cette composition, la couche sensible est élaborée à partir d'une formulation sol-gel composée de 0,33 TEOS (orthosilicate de tétraéthyle) ; 0,66 Ph-TEOS (phényl-triéthoxysilane) ; 7,8 $H_2O$ ; 0,07 HCl ; 6,2 PrOH (isopropanol), les différentes valeurs indiquées correspondant à des proportions molaires.

Plus particulièrement, les courbes 700, 710, 720, 730, 740, et 750 représentées respectivement aux figures 7A à 7F représentent la variation de l'indice de réfraction de l'élément réflecteur sensible 3 de composition S3M3P3 en fonction de la température de cette couche sensible 33, lorsqu'elle a été exposée respectivement et de manière individuelle aux composés volatils suivants :

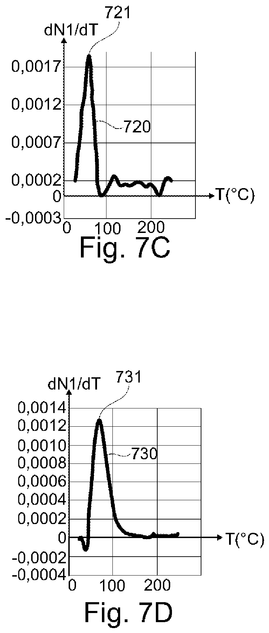

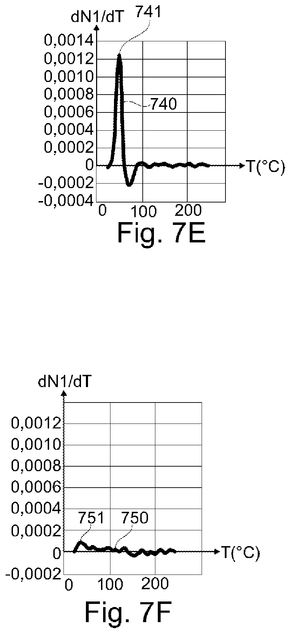

* hexane (figure 7A),
* toluène (figure 7B),
* acétylacétone (figure 7C),
* chloroforme (figure 7D),
* éthylène glycol (figure 7E),
* eau (figure 7F).

Chacune de ces courbes 700-750 présente un (ou plusieurs) pic de variation de l'indice de réfraction de l'élément réflecteur sensible 3 plus ou moins marqué. En particulier pour l'eau, le pic de variation 751 est très faible. Ce pic de variation 751 est détecté pour une température de la couche sensible 33 de 40°C environ. Cette analyse spectrale permet de déduire que la couche sensible 33 de composition S3M3P3 présente un caractère hydrophobe car elle capte très peu d'eau et l'eau désorbe très facilement (température de désorption de 40°C).

Pour les autres composés volatils A, B, C, qui eux sont de nature organique, les variations d'indice de réfraction de l'élément réflecteur sensible 3 sont plus marquées.

Selon la courbe 710 de la figure 7B, un pic de variation 711 marqué se produit pour une température de la couche sensible 33 de 53°C environ, correspondant à la désorption du toluène de la couche sensible 33.

Selon la courbe 720 de la figure 7C, un pic de variation 721 marqué se produit pour une température de la couche sensible 33 de 59°C environ, correspondant à la désorption de l'acétylacétone de la couche sensible 33.

Selon la courbe 730 de la figure 7D, un pic de variation 731 marqué se produit à une température de la couche sensible 33 de 75°C, correspondant à la désorption du chloroforme de la couche sensible 33.

Selon la courbe 740 de la figure 7E, un pic de variation 741 marqué se produit à une température de la couche sensible 33 de 53°C, correspondant à la désorption de l'éthylène glycol de la couche sensible 33.

Les températures correspondant à ces différents pics correspondent aux températures pour lesquelles cette courbe présente une dérivée nulle. Il est ainsi possible de déterminer aisément la nature du composé volatil désorbé en fonction de sa température de désorption de la couche sensible 33. Ces valeurs peuvent par exemple permettre la constitution d'une table pour différents composés volatils A, B, C afin de connaître leur température de désorption de la couche sensible 33 et de faciliter leur identification pour une couche sensible 33 de composition chimique donnée.

La courbe 700 de la figure 7A présente un premier 701, un deuxième 702 et un troisième 703 pics de variation de l'indice de réfraction de la couche sensible 33. Ces premier 701, deuxième 702, et troisième 703 pics de variations correspondent à la désorption différenciée de différents isomères de l'hexane et se produisent à des températures différentes qui sont respectivement de 69°C, 100°C et 179°C. Ces différents isomères de l'hexane peuvent correspondre au n-hexane, au 2-méthylpentane et au 3-méthylpentane par exemple. Ainsi, il est possible de différencier différents isomères d'un même composé chimique en fonction de sa température de désorption de la couche sensible 33.

En revenant sur les courbes 710 et 740 représentées respectivement en référence aux figures 7B et 7E, on remarque que le toluène et l'éthylène glycol présentent la même température de désorption pour la couche sensible 33 de composition S3M3P3. Cette couche sensible 33 de composition S3M3P3 n'est donc pas adaptée pour permettre la détection de ces composés volatils s'ils sont en mélange dans l'atmosphère à tester car leurs pics de variation 711 et 741 sont confondus. Dans une telle éventualité, il peut être souhaitable d'utiliser un élément réflecteur sensible 3 comprenant une pluralité de couches sensibles 33a, 33b, 33c comme représenté en référence à la figure 3B afin de permettre notamment la discrimination de ces deux composés volatils. Cette discrimination peut être réalisée soit en utilisant des couches sensibles 33a, 33b, 33c présentant des affinités chimiques perpendiculaires pour ces deux composés volatils, ou encore des couches sensibles 33a, 33b, 33c pour lesquelles ces deux composés volatils présentent des températures de désorption différentes comme cela est décrit ci-après.

Incidence de la composition chimique de la couche sensible :

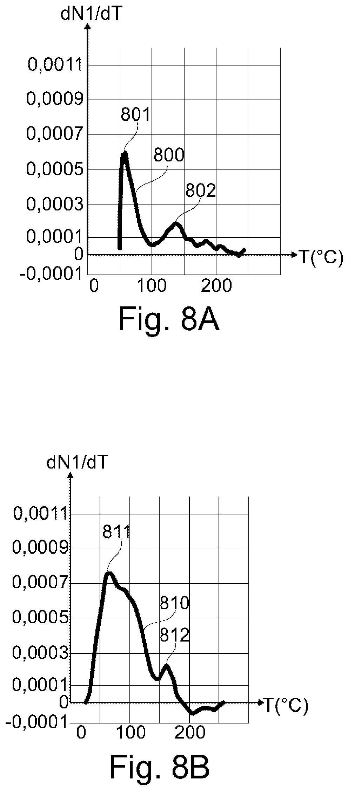

Afin de justifier de la différence des températures de désorption des composés volatils A, B, C en fonction de la composition chimique de la couche sensible 33 et de palier à l'éventualité d'avoir des composés volatils différents qui sont désorbés de la couche sensible 33 à la même température, comme cela est le cas pour le toluène et l'éthylène glycol dans l'exemple précédent, les inventeurs ont exposés des couches sensibles 33 de différentes compositions chimiques à une même atmosphère comprenant des composés volatils A, B, C. Les résultats des analyses de désorption thermique sont représentés en référence aux figures 8A à 8C. Plus particulièrement, les inventeurs ont préparé trois couches sensibles de compositions chimiques différentes, intitulées : REF, S3P7 et S3M3P3. Ces différentes couches sensibles ont été élaborées à partir de formulation sol-gel de compositions chimiques suivantes, les valeurs numériques données correspondant à des proportions molaires.

Composition REF : 1 TEOS (orthosilicate de tétraéthyle) ; 4 $H_2O$ ; 0,17 HCl ; 5,8 PrOH (isopropanol) ;
Composition S3P7 : 0,33 TEOS (orthsilicate de tétraéthyle) ; 0,33 MTEOS (triéthoxyméthylsilane) ; 0,33 Ph-TEOS (phényl-triéthoxysilane) ; 7,8 $H_2O$ ; 0,07 HCl ; 6,2 PrOH (isopropanol) ;
Composition S3M3P3 : 0,33 TEOS (orthosilicate de tétraéthyle) ; 0,66 Ph-TEOS (phényl-triéthoxysilane) ; 7,8 $H_2O$ ; 0,07 HCl ; 6,2 PrOH (isopropanol).

D'autre part, l'atmosphère à laquelle ces différentes couches sensibles 33 ont été exposées simultanément correspond, pour cet exemple particulier, à l'habitacle d'un véhicule automobile pendant une durée de 5 jours.

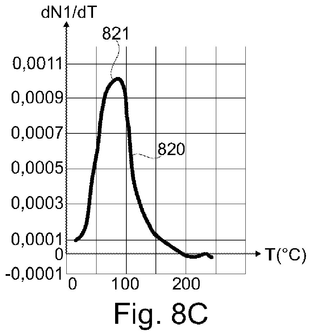

Plus précisément, la figure 8A représente la courbe 800 de variation de l'indice de réfraction de l'élément réflecteur sensible 3 présentant la couche sensible 33 de composition REF, la figure 8B représente la courbe 810 de variation de l'indice de réfraction de l'élément réflecteur sensible 3 présentant la couche sensible 33 de composition S3P7, et la figure 8C représente la courbe 820 de variation de l'indice de réfraction de l'élément réflecteur sensible 33 présentant la couche sensible 33 de composition S3M3P3. Plus particulièrement, ces courbes 800, 810 et 820 correspondent aux variations de l'indice de réfraction de l'élément réflecteur sensible 3 en fonction de la température de la couche sensible 33.

Selon ces différentes figures 8A à 8C, on remarque que la courbe 820 de la figure 8C présente un unique pic de variation 821 de l'indice de réfraction de l'élément réflecteur sensible 3 fortement marqué pour une température de la couche sensible 33 de 86°C environ alors que les autres courbes présentent deux pics de variations 801, 802, 811, 812 de l'indice de réfraction de l'élément réflecteur sensible 3 en fonction de la température. On peut ainsi considérer que la couche sensible 33 de composition S3M3P3 adsorbe un unique composé volatil alors que les couches sensibles 33 de composition REF et S3P7 adsorbent deux composés volatils présents dans l'atmosphère de cet habitacle de véhicule automobile. Cela montre une sélectivité de la couche sensible 33 selon sa composition chimique à certains composés volatils A, B, C.

Par ailleurs, la courbe 800 de la figure 8A présente deux pics de variation 801, 802 de l'indice de réfraction de l'élément réflecteur sensible 3 pour des températures de la couche sensible 33 respectivement de 55°C environ et de 132°C environ, correspondant à la désorption respectivement de deux composés volatils de la couche sensible 33 de composition chimique REF. D'autre part, la courbe 810 de la figure8B présente deux pics de variation 811, 812 de l'indice de réfraction de l'élément réflecteur sensible 3 pour des températures de la couche sensible 33 respectivement de 70°C environ et 160°C environ, correspondant à la désorption respectivement de deux composés volatils de la couche sensible de composition chimique S3P7. On peut supposer que ces deux pics de variations 801, 802, 811, 812 de l'indice de réfraction des couches sensibles 33 représentés en référence aux figures 8A et 8B correspondent à la désorption de deux composés volatils A, B identiques. La variation de la température de désorption de ces composés volatils en fonction de la composition chimique de la couche sensible 33 permet d'avoir une dimension additionnelle à la sélectivité du dispositif optique de détection 1 qui combinerait plusieurs couches sensibles 33a, 33b, 33c comme décrit en référence à la figure 3B. Il est ainsi possible de réaliser des mesures plus sensibles et de permettre une discrimination de ces composés volatils A, B, C s'ils présentaient une même température de désorption pour une couche sensible 33 de composition unique comme cela est le cas pour le toluène et l'éthylène glycol dans le cas de la couche sensible 33 de composition S3M3P3 décrit précédemment.

Les différents modes de réalisation décrits ci-dessus sont des exemples donnés à titre illustratif et non limitatif. En effet, il est tout à fait possible pour l'homme de l'art d'adapter la loi de chauffe de la couche sensible 33, l'épaisseur e de la couche sensible 33, l'épaisseur s de la couche conductrice d'électricité 35, la composition des couches de support 31, sensible 33, ou conductrice d'électricité 35 selon ses besoins sans sortir du cadre de la présente description. D'autre part, l'homme de l'art pourra utiliser des sources lumineuses présentant des longueurs d'ondes différentes de celles décrites dans les présents exemples sans sortir du cadre de la présente invention. De plus, les indices de réfraction des différentes couches de l'élément réflecteur sensible 3 peuvent être modifiée par l'homme de l'art en fonction des besoins sans sortir du cadre de la présente invention.

Ainsi, l'obtention d'un détecteur de composés volatils A, B, C simple d'utilisation, bon marché, efficace même pour de faibles concentrations de composés volatils A, B, C dans l'atmosphère à tester et offrant des résultats de mesure dans un temps court est possible grâce au dispositif optique de détection 1 décrit précédemment et plus particulièrement à l'élément réflecteur sensible 3 mis en oeuvre dans le procédé de détection et de quantification 100 décrit ci-dessus.

**Revendications**

1. Dispositif optique de détection (1) de composés volatils (A, B, C), **caractérisé en ce qu'**il comporte :

   - un élément réflecteur sensible (3) dont l'intensité lumineuse réfléchie varie en fonction des composés volatils (A, B, C) contenus dans une atmosphère à tester et de leur concentration, l'élément réflecteur sensible (3) comportant :

      * un substrat (31),
      * au moins une couche sensible (33) transparente entre 400 nm et 1000 nm et poreuse configurée pour permettre l'adsorption et la désorption des composés volatils (A, B, C) contenus dans l'atmosphère à tester, et
      * une couche conductrice d'électricité (35) prise en sandwich entre la couche de substrat (31) et la couche sensible (33) et disposée directement au contact de la couche de substrat (31) et de la couche sensible (33), ladite couche conductrice d'électricité (35) étant configurée pour permettre la chauffe de la couche sensible (33) par effet Joule afin de permettre la désorption des composés volatils (A, B, C) adsorbés sur la couche sensible (33),

   - au moins une source de lumière (5) monochromatique ou quasi-monochromatique disposée pour éclairer la couche sensible (33) sous un angle incident ($\alpha$),
   - au moins un détecteur de lumière (7) configuré pour mesurer l'intensité lumineuse réfléchie par l'élément réflecteur sensible (3) sous un angle de détection ($\beta$) la couche conductrice d'électricité (35) étant transparente et non diffusante entre 400 nm et 1000 nm de façon à ne pas nuire à l'intensité lumineuse réfléchie par l'élément réflecteur sensible (3) sous l'angle de détection, et
   - une unité de traitement et de calcul (9) configurée pour déterminer les composés volatils (A, B, C) présents dans l'atmosphère à tester selon leurs températures de désorption et leur concentration par variation de l'intensité lumineuse réfléchie par l'élément réflecteur sensible (3).

2. Dispositif optique de détection (1) selon la revendication précédente, **caractérisé en ce que** la présence d'un ou de plusieurs composés volatils (A, B, C) désorbés est détectée par comparaison de l'intensité lumineuse réfléchie par l'élément réflecteur (3) sans composé volatil adsorbé à une température donnée avec l'intensité lumineuse réfléchie par l'élément réflecteur (3) juste après désorption à cette même température.

3. Dispositif optique de détection (1) selon l'une quelconque des revendications précédentes, **caractérisé en ce que** la concentration du ou des composés volatils (A, B, C) détectés dans l'atmosphère à tester est déterminée par intégration d'une surface de pics de variation de l'intensité lumineuse réfléchie correspondant à la désorption du ou des composés volatils (A, B, C).

4. Dispositif optique de détection (1) selon l'une quelconque des revendications précédentes, **caractérisé en ce que** l'au moins une couche sensible (33) présente une épaisseur (e) comprise entre 50 nm et 2000 nm, notamment comprise entre 400 nm et 1200 nm, et plus particulièrement entre 500 nm et 800 nm.

5. Dispositif de détection optique (1) selon l'une quelconque des revendications précédentes, **caractérisé en ce que** l'au moins une couche sensible (33) présente une taille moyenne de pores inférieure à 2 nm.

6. Dispositif de détection optique (1) selon l'une quelconque des revendications précédentes, **caractérisé en ce que** l'au moins une couche sensible (33) présente une porosité inférieure à 25 %.

7. Dispositif de détection optique (1) selon l'une quelconque des revendications précédentes, **caractérisé en ce que** l'au moins une couche sensible (33) est réalisée en silice sol-gel ou par un xérogel.

8. Dispositif de détection optique (1) selon l'une quelconque des revendications précédentes, **caractérisé en ce que** l'au moins une couche sensible (33) présente un indice de réfraction compris entre 1,2 et 1,6, notamment entre 1,3 et 1,5, pour les longueurs d'ondes comprises entre 400 nm et 1000 nm avant son exposition à l'atmosphère à tester.

9. Dispositif de détection optique (1) selon l'une quelconque des revendications précédentes, **caractérisé en ce que** l'au moins une couche sensible (33) présente une structuration configuré pour augmenter les variations du signal optique.

**10.** Dispositif de détection optique (1) selon l'une quelconque des revendications précédentes, **caractérisé en ce que** l'élément réflecteur sensible (3) comporte une ou plusieurs couches sensibles (33).

**11.** Dispositif de détection optique (1) selon l'une quelconque des revendications précédentes, **caractérisé en ce que** l'angle incident ($\alpha$) et l'angle de détection ($\beta$) sont respectivement compris entre 30° et 75° par rapport à une normale (11) de le couche sensible (33).

**12.** Dispositif de détection optique (1) selon l'une quelconque des revendications précédentes, **caractérisé en ce que** la couche conductrice d'électricité (35) présente une épaisseur (s) inférieure ou égale à 150 nm, notamment comprise entre 70 nm et 90 nm.

**13.** Dispositif de détection optique (1) selon l'une quelconque des revendications précédentes, **caractérisé en ce que** la couche conductrice d'électricité (35) présente une résistivité comprise entre $10^{-4}$ $\Omega$.m et $10^{-7}$ $\Omega$.m de manière à permettre la chauffe de la couche sensible (33) par effet Joule.

**14.** Dispositif de détection optique (1) selon l'une quelconque des revendications précédentes, **caractérisé en ce que** la couche conductrice d'électricité (35) est réalisée en oxyde conducteur transparent, notamment en oxyde d'indium dopé à l'étain (ITO), en oxyde de zinc dopé à l'aluminium (AZO), en oxyde de zinc dopé au gallium (GZO), ou en oxyde d'étain dopé au fluor (FTO).

**15.** Procédé de détection et de quantification (100) de composés volatils (A, B, C) dans une atmosphère à tester mettant en oeuvre un dispositif de détection optique (1) selon l'une quelconque des revendications précédentes, **caractérisé en ce qu'**il comprend les étapes suivantes :

- éclairage (E1) de la couche sensible (33) sous un angle incident ($\alpha$) avec la source de lumière (5) monochromatique ou quasi-monochromatique et mesure de l'intensité lumineuse réfléchie par l'élément réflecteur sensible (3),
- exposition (E2) de l'élément réflecteur sensible (3) à l'atmosphère à tester pendant une durée prédéterminée de manière à permettre l'adsorption de composés volatils (A, B, C) contenus dans cette atmosphère à tester sur la couche sensible (33) de l'élément réflecteur sensible (3),
- chauffe (E3) de la couche sensible (33) par la couche conductrice d'électricité (35) afin de permettre une désorption contrôlée des composés volatils (A, B, C) de la couche sensible (33),
- mesure de l'intensité lumineuse réfléchie (E4) par l'élément réflecteur sensible (3) au cours de l'étape de chauffe (E3) afin de déterminer une variation de l'indice de réfraction de l'élément réflecteur sensible (3), et
- suivi de l'évolution (E5) de l'indice de réfraction de l'élément réflecteur sensible (3) afin de déterminer la nature et la quantité de composé volatil (A, B, C) désorbé de la couche sensible (33) lors de l'étape de chauffe (E3), ladite étape de suivi de l'évolution (E5) de l'indice de réfraction de l'élément réflecteur sensible (3) étant réalisée par l'unité de traitement et de calcul (9).

**16.** Procédé de détection et de quantification (100) de composés volatils (A, B, C) dans une atmosphère à tester selon la revendication 15, **caractérisé en ce que** l'étape de chauffe (E3) de la couche sensible (33) est réalisée par une chauffe contrôlée suivant une loi de chauffe comprise entre 1°C/s et 20°C/s.

**17.** Procédé de détection et de quantification (100) de composés volatils (A, B, C) dans une atmosphère à tester selon l'une quelconque des revendications 15 ou 16, **caractérisé en ce que** la détermination de la nature du composé volatil (A, B, C) désorbé au cours de l'étape de chauffe (E3) est réalisée par traitement mathématique différentiel des courbes d'évolution de la réponse optique en fonction de la température.

**18.** Procédé de détection et de quantification (100) de composés volatils (A, B, C) dans une atmosphère à tester selon l'une quelconque des revendications 15 à 17, **caractérisé en ce que** la quantification de composé volatil (A, B, C) désorbé est réalisée par intégration d'une surface d'un pic de variation de l'intensité lumineuse réfléchie par l'élément réflecteur sensible (3) correspondant à la désorption du composé volatil correspondant.

**19.** Procédé de détection et de quantification (100) de composés volatils (A, B, C) dans une atmosphère à tester selon l'une quelconque des revendications 15 à 18, **caractérisé en ce que** l'identification du composé volatil (A, B, C), dans le cas où il y a mélange de composés volatils (A, B, C), est réalisée par déconvolution de l'intensité de lumière réfléchie par l'élément réflecteur sensible (33) lors de la désorption de ce gaz.

**Patentansprüche**

1. Optische Detektionsvorrichtung (1) zur Detektion von flüchtigen Verbindungen (A, B, C), **dadurch gekennzeichnet, dass** sie umfasst:

   - ein empfindliches reflektierendes Element (3), dessen reflektierte Lichtstärke in Abhängigkeit von den in einer zu testenden Atmosphäre enthaltenen flüchtigen Verbindungen (A, B, C) und von ihrer Konzentration variiert, wobei das empfindliche reflektierende Element (3) umfasst:

      * ein Substrat (31),
      * mindestens eine empfindliche Schicht (33), die zwischen 400 nm und 1000 nm transparent ist und porös ist und dazu ausgestaltet ist, die Adsorption und die Desorption der in der zu testenden Atmosphäre enthaltenen flüchtigen Verbindungen (A, B, C) zu ermöglichen, und
      * eine Elektrizität leitende Schicht (35), die sandwichartig zwischen der Substratschicht (31) und der empfindlichen Schicht (33) aufgenommen ist und direkt in Kontakt mit der Substratschicht (31) und der empfindlichen Schicht (33) angeordnet ist, wobei die Elektrizität leitende Schicht (35) dazu ausgestaltet ist, das Erwärmen der empfindlichen Schicht (33) durch Joule-Effekt zu ermöglichen, um die Desorption der auf der empfindlichen Schicht (33) adsorbierten flüchtigen Verbindungen (A, B, C) zu ermöglichen,

   - mindestens eine monochromatische oder quasi-monochromatische Lichtquelle (5), die angeordnet ist, um die empfindliche Schicht (33) unter einem Einfallswinkel ($\alpha$) zu beleuchten,
   - mindestens einen Lichtdetektor (7), der dazu ausgestaltet ist, die von dem empfindlichen reflektierenden Element (3) unter einem Detektionswinkel ($\beta$) reflektierte Lichtstärke zu messen, wobei die Elektrizität leitende Schicht (35) zwischen 400 nm und 1000 nm transparent ist und nicht streuend ist, so dass die von dem empfindlichen reflektierenden Element (3) unter dem Detektionswinkel reflektierte Lichtstärke nicht beeinträchtigt wird, und
   - eine Verarbeitungs- und Recheneinheit (9), die dazu ausgestaltet ist, die in der zu testenden Atmosphäre vorhandenen flüchtigen Verbindungen (A, B, C) gemäß ihren Desorptionstemperaturen und ihrer Konzentration durch Variation der von dem empfindlichen reflektierenden Element (3) reflektierten Lichtstärke zu bestimmen.

2. Optische Detektionsvorrichtung (1) nach dem vorhergehenden Anspruch, **dadurch gekennzeichnet, dass** das Vorhandensein einer oder mehrerer desorbierter flüchtiger Verbindungen (A, B, C) durch Vergleichen der Lichtintensität, die von dem reflektierenden Element (3) ohne adsorbierte flüchtige Verbindung bei einer gegebenen Temperatur reflektiert wird, mit der Lichtintensität, die von dem reflektierenden Element (3) unmittelbar nach Desorption bei derselben Temperatur reflektiert wird, detektiert wird.

3. Optische Detektionsvorrichtung (1) nach einem der vorhergehenden Ansprüche, **dadurch gekennzeichnet, dass** die Konzentration der in der zu testenden Atmosphäre detektierten flüchtigen Verbindung(en) (A, B, C) durch Integration einer Fläche von Peaks der Variation der Lichtstärke bestimmt wird, die der Desorption der flüchtigen Verbindung(en) (A, B, C) entsprechen.

4. Optische Detektionsvorrichtung (1) nach einem der vorhergehenden Ansprüche, **dadurch gekennzeichnet, dass** die mindestens eine empfindliche Schicht (33) eine Dicke (e) zwischen 50 nm und 2000 nm, insbesondere zwischen 400 nm und 1200 nm und noch bevorzugter zwischen 500 nm und 800 nm aufweist.

5. Optische Detektionsvorrichtung (1) nach einem der vorhergehenden Ansprüche, **dadurch gekennzeichnet, dass** die mindestens eine empfindliche Schicht (33) eine mittlere Porengröße von weniger als 2 nm aufweist.

6. Optische Detektionsvorrichtung (1) nach einem der vorhergehenden Ansprüche, **dadurch gekennzeichnet, dass** die mindestens eine empfindliche Schicht (33) eine Porosität von weniger als 25 % aufweist.

7. Optische Detektionsvorrichtung (1) nach einem der vorhergehenden Ansprüche, **dadurch gekennzeichnet, dass** die mindestens eine empfindliche Schicht (33) aus Sol-Gel-Siliciumdioxid oder mit einem Xerogel ausgeführt ist.

8. Optische Detektionsvorrichtung (1) nach einem der vorhergehenden Ansprüche, **dadurch gekennzeichnet, dass** die mindestens eine empfindliche Schicht (33) einen Brechungsindex zwischen 1,2 und 1,6, insbesondere zwischen 1,3 und 1,5, bei Wellenlängen zwischen 400 nm und 1000 nm vor ihrer Exposition gegenüber der zu testenden Atmosphäre aufweist.

9. Optische Detektionsvorrichtung (1) nach einem der vorhergehenden Ansprüche, **dadurch gekennzeichnet, dass** die mindestens eine empfindliche Schicht (33) eine Strukturierung aufweist, die dazu ausgestaltet ist, die Variationen des optischen Signals zu verstärken.

10. Optische Detektionsvorrichtung (1) nach einem der vorhergehenden Ansprüche, **dadurch gekennzeichnet, dass** das empfindliche reflektierende Element (3) eine oder mehrere empfindliche Schichten (33) umfasst.

11. Optische Detektionsvorrichtung (1) nach einem der vorhergehenden Ansprüche, **dadurch gekennzeichnet, dass der Einfallswinkel ($\alpha$) und der Detektionswinkel ($\beta$)** jeweils zwischen 30° und 75° in Bezug auf eine Senkrechte (11) zu der empfindlichen Schicht (33) betragen.

12. Optische Detektionsvorrichtung (1) nach einem der vorhergehenden Ansprüche, **dadurch gekennzeichnet, dass** die Elektrizität leitende Schicht (35) eine Dicke (s) kleiner oder gleich 150 nm, insbesondere zwischen 70 nm und 90 nm aufweist.

13. Optische Detektionsvorrichtung (1) nach einem der vorhergehenden Ansprüche, **dadurch gekennzeichnet, dass** die Elektrizität leitende Schicht (35) einen spezifischen Widerstand zwischen $10^{-4}$ $\Omega$**.m und $10^{-7}$ $\Omega$.m aufweist, so dass sie das** Erwärmen der empfindlichen Schicht (33) durch Joule-Effekt ermöglicht.

14. Optische Detektionsvorrichtung (1) nach einem der vorhergehenden Ansprüche, **dadurch gekennzeichnet, dass** die Elektrizität leitende Schicht (35) aus transparentem leitfähigem Oxid, insbesondere aus zinndotiertem Indiumoxid (ITO), aus aluminiumdotiertem Zinkoxid (AZO, aus galliumdotiertem Zinkoxid (GZO) oder aus fluordotiertem Zinnoxid (FTO) ausgeführt ist.

15. Verfahren zur Detektion und Quantifizierung (100) von flüchtigen Verbindungen (A, B, C) in einer zu testenden Atmosphäre, bei dem eine optische Detektionsvorrichtung (1) nach einem der vorhergehenden Ansprüche eingesetzt wird, **dadurch gekennzeichnet, dass** es die folgenden Schritte umfasst:

   - Beleuchten (E1) der empfindlichen Schicht (33) unter einem Einfallswinkel ($\alpha$) mit der monochromatischen oder quasi-monochromatischen Lichtquelle (5) und Messen der von dem empfindlichen reflektierenden Element (3) reflektierten Lichtstärke,
   - Exposition (E2) des empfindlichen reflektierenden Elements (3) gegenüber der zu testenden Atmosphäre während einer vorbestimmten Dauer, so dass die Adsorption der in dieser zu testenden Atmosphäre enthaltenen flüchtigen Verbindungen (A, B, C) auf der empfindlichen Schicht (33) des empfindlichen reflektierenden Elements (3) ermöglicht wird,
   - Erwärmen (E3) der empfindlichen Schicht (33) durch die Elektrizität leitende Schicht (35), um eine kontrollierte Desorption der flüchtigen Verbindungen (A, B, C) aus der empfindlichen Schicht (33) zu ermöglichen,
   - Messen (E4) der von dem empfindlichen reflektierenden Element (3) im Laufe des Schritts des Erwärmens (E3) reflektierten Lichtstärke, um eine Variation des Brechungsindex des empfindlichen reflektierenden Elements (3) zu bestimmen, und
   - Nachverfolgen des Verlaufs (E5) des Brechungsindex des empfindlichen reflektierenden Elements (3), um die Art und die Menge der von der empfindlichen Schicht (33) beim Schritt des Erwärmens (E3) desorbierten flüchtigen Verbindung (A, B, C) zu bestimmen, wobei der Schritt des Nachverfolgens des Verlaufs (E5) des Brechungsindex des empfindlichen reflektierenden Elements (3) von der Verarbeitungs- und Recheneinheit (9) ausgeführt wird.

16. Verfahren zur Detektion und Quantifizierung (100) von flüchtigen Verbindungen (A, B, C) in einer zu testenden Atmosphäre nach Anspruch 15, **dadurch gekennzeichnet, dass** der Schritt des Erwärmens (E3) der empfindlichen Schicht (33) durch ein kontrolliertes Erwärmen nach einem Erwärmungsgesetz zwischen 1 °C/s und 20 °C/s ausgeführt wird.

17. Verfahren zur Detektion und Quantifizierung (100) von flüchtigen Verbindungen (A, B, C) in einer zu testenden Atmosphäre nach einem der Ansprüche 15 oder 16, **dadurch gekennzeichnet, dass** das Bestimmen der Art der flüchtigen Verbindung (A, B, C), die im Laufe des Schritts des Erwärmens (E3) desorbiert wird, durch mathematische Differentialverarbeitung der Verlaufskurven der optischen Antwort in Abhängigkeit von der Temperatur ausgeführt wird.

18. Verfahren zur Detektion und Quantifizierung (100) von flüchtigen Verbindungen (A, B, C) in einer zu testenden Atmosphäre nach einem der Ansprüche 15 bis 17, **dadurch gekennzeichnet, dass** die Quantifizierung der desorbierten flüchtigen Verbindung (A, B, C) durch Integration einer Fläche eines Peaks der Variation der von dem empfindlichen reflektierenden Element (3) reflektierten Lichtstärke bestimmt wird, der der Desorption der entsprechenden flüchtigen Verbindung entspricht.

19. Verfahren zur Detektion und Quantifizierung (100) von flüchtigen Verbindungen (A, B, C) in einer zu testenden Atmosphäre nach einem der Ansprüche 15 bis 18, **dadurch gekennzeichnet, dass** die Identifizierung der flüchtigen Verbindung (A, B, C) in dem Fall, in dem eine Mischung flüchtiger Verbindungen (A, B, C) vorliegt, durch Dekonvolution der von dem empfindlichen reflektierenden Element (33) bei der Desorption dieses Gases reflektierten Lichtstärke ausgeführt wird.

**Claims**

1. Optical device (1) for detecting volatile compounds (A, B, C), **characterized in that** it comprises:

   - a sensitive reflecting element (3) the reflected light intensity of which varies as a function of the volatile compounds (A, B, C) contained in an atmosphere to be tested and on their concentration, the sensitive reflecting element (3) comprising:

     ◦ a substrate (31),
     ◦ at least one sensitive layer (33) that is transparent between 400 nm and 1000 nm and porous, said sensitive layer being configured to allow volatile compounds (A, B, C) contained in the atmosphere to be tested to adsorb and desorb, and
     ◦ an electrically conductive layer (35) sandwiched between the substrate layer (31) and the sensitive layer (33) and placed directly in contact with the substrate layer (31) and with the sensitive layer (33), said electrically conductive layer (35) being configured to allow the sensitive layer (33) to be heated by Joule heating in order to allow the volatile compounds (A, B, C) adsorbed on the sensitive layer (33) to desorb,
     ◦ at least one monochromatic or quasi-monochromatic light source (5) placed to illuminate the sensitive layer (33) at an incident angle ($\alpha$),
     ◦ at least one light detector (7) configured to measure the light intensity reflected by the sensitive reflecting element (3) at a detection angle ($\beta$), the electrically conductive layer (35) being transparent and non-scattering between 400 nm and 1000 nm so as not to adversely affect the light intensity reflected by the sensitive reflecting element (3) at the detection angle, and
     ◦ a computing and processing unit (9) configured to determine the volatile compounds (A, B, C) present in the atmosphere to be tested depending on their desorption temperatures and their concentration via variation in the light intensity reflected by the sensitive reflecting element (3).

2. Optical detecting device (1) according to the preceding claim, **characterized in that** the presence of one or more desorbed volatile compounds (A, B, C) is detected via comparison of the light intensity reflected by the reflecting element (3) without any volatile compounds adsorbed at a given temperature with the light intensity reflected by the reflecting element (3) just after desorption at the same temperature.

3. Optical detecting device (1) according to either one of the preceding claims, **characterized in that** the concentration of the one or more volatile compounds (A, B, C) detected in the atmosphere to be tested is determined via integration of an area of peaks in variation of the reflected light intensity corresponding to the desorption of the one or more volatile compounds (A, B, C).

4. Optical detecting device (1) according to any one of the preceding claims, **characterized in that** the at least one sensitive layer (33) has a thickness (e) comprised between 50 nm and 2000 nm, notably comprised between 400 nm and 1200 nm, and more particularly between 500 nm and 800 nm.

5. Optical detecting device (1) according to any one of the preceding claims, **characterized in that** the at least one sensitive layer (33) has an average pore size smaller than 2 nm.

6. Optical detecting device (1) according to any one of the preceding claims, **characterized in that** the at least one sensitive layer (33) has a porosity lower than 25%.

7. Optical detecting device (1) according to any one of the preceding claims, **characterized in that** the at least one sensitive layer (33) is made of sol-gel silica or of a xerogel.

8. Optical detecting device (1) according to any one of the preceding claims, **characterized in that** the at least one sensitive layer (33) has a refractive index comprised between 1.2 and 1.6, and notably between 1.3 and 1.5, at wavelengths comprised between 400 nm and 1000 nm before its exposure to the atmosphere to be tested.

9. Optical detecting device (1) according to any one of the preceding claims, **characterized in that** the at least one sensitive layer (33) has a structure configured to increase the variations in the optical signal.

10. Optical detecting device (1) according to any one of the preceding claims, **characterized in that** the sensitive reflecting element (3) comprises one or more sensitive layers (33).

11. Optical detecting device (1) according to any one of the preceding claims, **characterized in that** the incident angle ($\alpha$) and the detection angle ($\beta$) are respectively comprised between 30° and 75° with respect to a normal (11) to the sensitive layer (33).

12. Optical detecting device (1) according to any one of the preceding claims, **characterized in that** the electrically conductive layer (35) has a thickness (s) smaller than or equal to 150 nm, and notably comprised between 70 nm and 90 nm.

13. Optical detecting device (1) according to any one of the preceding claims, **characterized in that** the electrically conductive layer (35) has a resistivity comprised between $10^{-4}$ $\Omega$.m and $10^{-7}$ $\Omega$.m so as to allow the sensitive layer (33) to be heated by Joule heating.

14. Optical detecting device (1) according to any one of the preceding claims, **characterized in that** the electrically conductive layer (35) is made of a transparent conductive oxide, and notably of indium-doped tin oxide (ITO), aluminium-doped zinc oxide (AZO), gallium-doped zinc oxide (GZO), or fluorine-doped tin oxide (FTO).

15. Method (100) for detecting and quantifying volatile compounds (A, B, C) in an atmosphere to be tested implementing an optical detecting device (1) according to any one of the preceding claims, **characterized in that** it comprises the following steps:

- illuminating (E1) the sensitive layer (33) at an incident angle ($\alpha$) with the monochromatic or quasi-monochromatic light source (5) and measuring the light intensity reflected by the sensitive reflecting element (3),
- exposing (E2) the sensitive reflecting element (3) to the atmosphere to be tested for a predetermined time so as to allow volatile compounds (A, B, C) contained in this atmosphere to be tested to adsorb on the sensitive layer (33) of the sensitive reflecting element (3),
- heating (E3) the sensitive layer (33) via the electrically conductive layer (35) in order to allow the volatile compounds (A, B, C) to be controllably desorbed from the sensitive layer (33),
- measuring (E4) the light intensity reflected by the sensitive reflecting element (3) during the heating step (E3) in order to determine a variation in the refractive index of the sensitive reflecting element (3), and
- monitoring (E5) the variation in the refractive index of the sensitive reflecting element (3) in order to determine the nature and amount of volatile compound (A, B, C) desorbed from the sensitive layer (33) in the heating step (E3), said step (E5) of monitoring the variation in the refractive index of the sensitive reflecting element (3) being carried out by the computing and processing unit (9).

16. Method (100) for detecting and quantifying volatile compounds (A, B, C) in an atmosphere to be tested according to Claim 15, **characterized in that** the step (E3) of heating the sensitive layer (33) is carried out via controlled heating at a heating rate comprised between 1°C/s and 20°C/s.

17. Method (100) for detecting and quantifying volatile compounds (A, B, C) in an atmosphere to be tested according to either one of Claims 15 and 16, **characterized in that** the nature of the volatile compound (A, B, C) desorbed during the heating step (E3) is determined via differential mathematical processing of the curves of variation in the optical response as a function of temperature.

18. Method (100) for detecting and quantifying volatile compounds (A, B, C) in an atmosphere to be tested according to any one of Claims 15 to 17, **characterized in that** the desorbed volatile compound (A, B, C) is quantified via integration of an area of a peak of variation in the light intensity reflected by the sensitive reflecting element (3) corresponding to the desorption of the corresponding volatile compound.

19. Method (100) for detecting and quantifying volatile compounds (A, B, C) in an atmosphere to be tested according to any one of Claims 15 to 18, **characterized in that** the volatile compound (A, B, C), in the case where there is a mixture of volatile compounds (A, B, C), is identified via deconvolution of the light intensity reflected by the sensitive reflecting element (33) during the desorption of this gas.

Fig. 1

Fig. 2

Fig. 3A

Fig. 3B

Fig. 4A

Fig. 4B

Fig. 4C

Fig. 5

Fig. 6A

Fig. 6B

dN1/dT

## Fig. 7A

dN1/dT

## Fig. 7B

Fig. 7C

Fig. 7D

Fig. 7E

Fig. 7F

Fig. 8A

Fig. 8B

Fig. 8C

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2014189758 A
- WO 2007019517 A
- US 20160084786 A
- FR 2871573

- FR 3046244
- US 2014021967 A1
- US 9007593 B2

**Littérature non-brevet citée dans la description**

- Planar indium tin oxide heater for improved thermal distribution for métal oxide micromachined gas sensors. *Sensors,* 2016, vol. 16, 1612

- Porosity and mechanical properties of mesoporous thin films assessed by environmental ellipsometric porosimetry. **CEDRIC BOISSIÈRE et al.** Langmuir: the ACSjournal ofsurfaces and colloids. American Chemical Society, 2005, vol. 21, 12362-71